# EUROPEAN PATENT SPECIFICATION

(11) **EP 0 958 305 B1**
(45) Date of publication and mention of the grant of the patent: **04.06.2008**
(21) Application number: 97935511.2
(22) Date of filing: 04.07.1997
(51) Int. Cl.: C07K 14/00

(54) **INHIBITIONS OF THE INTERACTION BETWEEN P53 AND MDM2**
HEMMER DER INTERAKTION ZWISCHEN P53 UND MDM2
INHIBITEURS DE L'INTERACTION ENTRE p53 ET MDM2

(30) Priority: 05.07.1996 GB 9614197; 07.04.1997 GB 9707041
(43) Date of publication of application: 24.11.1999
(60) Divisional application: 06006683.4
(73) Proprietor: Cancer Research Technology Limited, London WC2A 3NL (GB)
(72) Inventor: LANE, David, By Saint Andrews, Fife KY16 0AL (GB); BÖTTGER, Volker, D-82110 Germering-Unterpfaffenhofen (DE); BÖTTGER, Angelika, D-82110 Germering-Unterpfaffenhofen (DE); PICKSLEY, Stephen, Bradford, West Yorkshire BD10 0BD (GB); HOCHKEPPEL, Heinz-Kurt, CH-4147 Aesch (CH); GARCIA-ECHEVERRIA, Carlos, CH-4052 Basel (CH); CHENE, Patrick, F-68100 Mulhouse (FR); FURET, Pascal, F-68800 Thann (FR)
(74) Representative: Kiddle, Simon John
(86) International application number: PCT/EP1997/003549
(87) International publication number: WO 1998/001467

(56) References cited:
- WO-A-93/20238
- WO-A-95/07934
- WO-A-96/02642
- S.M. PICKSLEY ET AL: "Immunochemical analysis of the interaction of p53 with MDM2" ONCOGENE, vol. 9, September 1994, pages 2523-2529, XP002058480
- J. LIN ET AL.: "Several hydrophobic amino acids in the p53 amino-terminal domain are required for transcriptional activation, binding to mdm-2 and the adenovirus 5 E1B 55-kD protein" GENES AND DEVELOPMENT, vol. 8, no. 10, May 1994, pages 1235-1246, XP002058481
- V. BÖTTGER ET AL: "Identification of novel mdm2 binding peptides by phage display" ONCOGENE, XP002058482
- H. LEE ET AL: "Molecular characterisation of Nos-A" J. BACTERIOL., vol. 173, no. 17, September 1991, pages 5606-5413, XP002058483
- KOSTER M. ET AL: 'Role for the outer membrane ferric siderophore receptor PupB in signal transduction across the bacterial cell envelope' THE EMBO JOURNAL vol. 13, no. 12, 1994, pages 2805 - 2813, XP000941419
- TONIOLO C.; BENEDETTI E.: 'Structures of Polypeptides from .alpha.-Amino Acids Disubstituted at the .alpha.-Carbon' MACROMOLECULES vol. 24, 1991, pages 4004 - 4009
- GARCIA-ECHEVERRIA C. ET AL: 'Discovery of Potent Antagonists of the Interaction between Human Double Minute 2 and Tumor Suppressor p53' JOURNAL OF MEDICINAL CHEMISTRY vol. 43, 2000, pages 3205 - 3208
- PANTANO M. ET AL: 'Synthesis and Conformational Analysis of (.alpha.Me)Leu/Aib Model Peptides' PEPTIDE RESEARCH vol. 6, no. 4, 1993, pages 196 - 204, XP008067680
- BASU G. ET AL: 'A Collective Motion Description of the 3-10/.alpha-Helix Transition: Implications for a Natural Reaction Coordinate' JOURNAL OF THE AMERICAN CHEMICAL SOCIETY vol. 116, 1994, pages 6307 - 6315
- WENSCHUH H. ET AL: 'Stepwise Automated Solid Phase Synthesis of Naturally Occurring Peptaibols Using FMOC Amino Acid Fluorides' JOURNAL OF ORGANIC CHEMISTRY vol. 60, 1995, pages 405 - 410
- KARLE I.L. ET AL: 'Accomodation of a D-Phe Residue into a Right-Handed 3-10-Helix: Structure of Boc-D-Phe-(Aib)4-Gly-L-Leu-(Aib)2-OMe, an Analogue of the Amino Terminal Segment of Antiamoebins and Emerimicins' BIOPOLYMERS vol. 33, 1993, pages 401 - 407
- CHENE P.: 'INHIBITING THE p53-MDM2 INTERACTION: AN IMPORTANT TARGET FOR CANCER THERAPY' NATURE REVIEWS vol. 3, February 2003, pages 102 - 109

## Description

The present invention relates to compounds capable of binding to the oncogene protein MDM2, processes for the preparation of such compounds, pharmaceutical preparations comprising such compounds, and uses of said compounds, e.g. in the therapeutic (including prophylactic) treatment of an animal or especially of the human body. The present further relates to methods of and compounds for inhibiting the growth of tumor cells which comprise the wild type p53 suppressor by interfering with the interaction between human p53 and human MDM2.

Inactivation of the p53 tumor suppressor is a frequent event in human neoplasia. Such inactivation of p53 may, for example, result from the binding of a cellular oncogene protein, such as MDM2. The protein encoded by the mdm2 gene, which is also referred to as hdm2 (human double minute 2) gene in the art, is capable of forming a complex with p53 both *in vitro* and *in vivo* and inhibit p53-mediated transactivation (J. Momand et al., Cell 69, 1237-1245 (1992)). Formation of this complex favors nucleoplasmic transformation because the complexed p53 essentially looses its tumor suppressor activity. MDM2 is overproduced in about 30 % of the human sarcomas and has been associated with an oncogenic phenotype. Compounds preventing or decreasing the binding of MDM2 to p53 alleviate the sequestration of p53, thus promoting p53 tumor suppressor activity. Surprisingly it has been found that the compounds of the invention interfere with the interaction of MDM2 with p53 and activate p53 function and p53 accumulation in normal cells having non-elevated MDM2 levels.

The MDM2 binding site is localized within the region of p53 represented approximately by amino acids 13 to 31 (PLSQETFSDLWKLLPENNV; single letter code) of mature human p53 protein. Recently, it has been found that peptide fragments of p53 which include the amino acid motif FxxLW wherein F, L, and W represent the single letter codes for amino acids phenylalanine, leucine and tryptophan, respectively, and X may be any amino acid, would be particularly suitable for interfering with the binding between p53 and MDM2 (Picksley et al., Oncogene 9, 2523-2529 (1994)) and WO 96/02642. However, there is still a need for compounds which are potent inhibitors of P53-MDM2 binding, and therefore beneficial in the treatment of p53-related diseases, such as (hyper)proliferative diseases. It is the object of the present invention to fulfill this and other needs.

In one aspect, the present invention is based on the surprising finding that a peptide with the phage consensus amino acid sequence P-X-F-X-D-Y-W-X-X-L, wherein X is any naturally occurring L-amino acid, and P, F, D, W and L represent the L-amino acids of proline (P), phenylalanine (F), aspartic acid (D), tyrosine (Y), tryptophan (W) and leucine (L), respectively, given in the single letter code, is capable of blocking the interaction of MDM2 with p53, as determinable e.g. in an ELISA assay, and shows a significant increase in specific blocking activity over the wildtype p53 peptide sequence.

As used herein, "mdm" refers to the oncogene and "MDM" refers to the protein obtainable as a result of expression of said gene. Even though in the strict sense "mdm" means "murine double minute gene2", as used herein it also refers to dm2 mutants, particularly interspecies mutants, such as hdm2 (human double minute gene2) in particular.

More specifically, it is an object of the present invention to provide compounds capable of interfering with the interaction between p53 and MDM2 and/or mdm2 in tumor cells having wild type p53, particularly human p53, and non-elevated MDM2 levels, as defined below, *in vivos* and *in vitro.* A preferred embodiment includes peptides and derivatives thereof, capable of binding to MDM2, particularly human DM2, and specifically inhibiting or blocking the binding of MDM2 to the p53 protein, particularly human p53, *in vitro* or *in vivo.* The peptides of the invention are better than the p53 wildtype peptide in inhibiting the hdm2 binding to p53 or a suitable p53 peptide, as can be determined e.g. in suitable ELISA-type assays, particularly the assays described in detail hereinafter, on the basis of the IC₅₀, i.e. the concentration of peptide necessary to inhibit the hdm2 or p53 binding by 50 %. The peptides of the invention mimic the MDM2 binding site on p53. The peptides provided herein consist of or comprise an amino acid motif (in N- to C-terminal order) of the formula

R₁-X₁-F-X₂-R₂-R₃-W-X₃-X₄-R₄ (I),

wherein
R₁ is a proline (P), leucine (L), glutamic acid (E), cysteine (C) or glutamine (Q),
X stands for one (any) natural amino acid,
R₂ is arginine (R), histidine (H), glutamic acid (E), cysteine (C), serine (S), or preferably aspartic acid (D),
R₃ is histidine (H), phenylalanine (F) or preferably tyrosine,
R₄ is phenylalanine (F), glutamine (Q) or preferably leucine (L); and
F and W (as well as the other capital letters given in brackets above) are used in accordance with the commonly used single letter code for amino acids and represent phenylalanine and tryptophan, respectively.

As used herein, the term "amino acid(s)" includes the free (charged or uncharged) form, or the monovalent or bivalent radical, the latter also being referred to as "amino acid residue". For example, in a 10mer peptide of formula (I), R₁ and R₄ are monovalent radicals, R₁ having a free amino group and R₄ having a free carboxy group, and X, for example, is a bivalent amino acid radical.

Preferred peptides of the invention consisting of or comprising the amino acid motif of formula (I) are peptides consisting of no more than fifteen amino acids (15mers), particularly 10mer, 11 mer, 12mer, 13mer, 14mer or 15mer peptides. In such peptides comprising the amino acid motif of formula (I) natural amino acid residues may be attached to the 10mer motif of formula (I) at the N-terminus, i.e. such additional amino acids precede R₁, at the N-terminus; at the C-terminus, i.e. such amino acids follow R₄; or at both ends of a peptide of formula (I). Sequences of exemplary 12mer and 15mer peptides are given e.g. in Example 8 hereinbelow.

As used herein, a natural amino acid is a natural α-amino acid having the L-configuration, such as those normally occurring in natural proteins. Unnatural amino acid refers to an amino acid, which normally does not occur in proteins, e.g. an epimer of a natural α-amino acid having the L-configuration, that is to say an amino acid having the unnatural D-configuration; or a (D,L)-isomeric mixture thereof; or a homologue of such an amino acid, for example a β-amino acid, an α,α-disubstituted amino acid, or an α-amino acid wherein the amino acid side chain has been shortened by one or two methylene groups or lengthened to up to 10 carbon atoms, such as an α-amino alkanoic acid with 5 up to and including 10 carbon atoms in a linear chain, an unsubstituted or substituted aromatic (α-aryl or α-aryl lower alkyl), for example a substituted phenylalanine or phenylglycine.

By selectively disrupting or preventing p53 from binding to MDM2 through its MDM2 binding site, the peptides of the invention, or derivatives thereof, can significantly decrease or avoid the negative regulatory effects of MDM2 on p53 activity. Therefore, the peptides, or derivatives thereof, of the invention can be used to restore p53 tumor suppressor function, e.g. in the treatment of tumor diseases or viral infections when enhanced activity of p53 is desired or required.

The peptide sequences of the invention show some homology to the sequence on p53 required for MDM2 binding, however, additional homologies are present which are absent from p53.

Preferred is a peptide of formula

R₁-X₁-F-X₂-R₂-R₃-W-X₃-X₄-R₄ (Ia),

wherein
R₁, R₂, R₃ and R₄ each have the meanings given for formula (I) above,
X₁ is arginine, asparagine, alanine, threonine or valine;
X₂ is methionine, isoleucine, threonine, arginine, alanine or serine;
X₃ is glutamic acid, threonine, alanine, phenylalanine or serine;
X₄ is glycine, glutamine, threonine, alanine or aspartic acid.

In particular, preferred peptides of the invention include the following (amino acid sequences are given in single letter code):

M-P-R-F-M-D-Y-W-E-G-L-N (II);

Q-P-T-F-S-D-Y-W-K-L-L-P (III)

P-R-P-A-C-V-F-A-D-Y-W-E-T-L-Y (IV).

As used herein, "peptide of the invention" refers to a linear compound comprising the amino acid motif of formula (I) and containing only natural amino acids which are linked by peptide bonds and which are in an unprotected form.

The present invention also provides derivatives of the peptides of the invention. Such derivatives may be linear or circular. Derivatives of the invention include molecules wherein a peptide of the invention is non-covalently or preferably covalently modified by substitution, chemical, enzymatic or other appropriate means with another atom or moiety including another peptide or protein. An example of a derivative comprising a peptide linked to another protein is exemplified by binding element TIP12/1 as described in example 10 below. The moiety may be "foreign" to a peptide of the invention as defined above in that it is an unnatural amino acid, or in that one or more, preferably one or two natural amino acid in the motif of formula (I) are replaced with another natural or unnatural amino acid as defined in the claims. Conjugates comprising a peptide or derivative of the invention covalently attached to another peptide or protein are also encompassed herein. Attachment of another moiety may involve a linker or spacer, e.g. an amino acid or peptidic linker. Derivatives of the invention also includes peptides wherein one, some or all potentially reactive groups, e.g. amino, carboxy, sulfhydryl or hydroxyl groups are in a protected form.

The atom or moiety derivatizing a peptide of the invention may serve analytical purposes, e.g. facilitate detection of the peptide of the invention, favor preparation or purification of the peptide, or improve a property of the peptide which is relevant for the purposes of the present invention. Such properties include e.g. cellular uptake, binding to MDM2, or suitability for in vivo administration, particularly solubility or stability against enzymatic degradation. Derivatives of the invention include a covalent or aggregative conjugate of a peptide of the invention with another chemical moiety, said derivative displaying essentially the same activity as the underivatized peptide of the invention, and a "peptide analogue" or "mimetic" which is modeled to resemble the three-dimensional structure of the amino acid motif of formula (I) and which is as defined in the claims. Examples of such mimetics are retro-inverso peptides (M. Chorev, M. Goodman, Acc. Chem. Res. 26, 266-273 (1993)). The designing of mimetics to a known pharmaceutically active compound is a known approach to the design of drugs based on a "lead" compound. This may be desirable e.g. where the "original" active compound is difficult or expensive to synthesize, or where it is unsuitable for a particular mode of administration, e.g. peptides are considered unsuitable active agents for oral compositions as they tend to be quickly degraded by proteases in the alimentary channel.

Examples of derivatives within the above general definitions are:
- Cyclic peptides or derivatives including compounds with a disulfide bridge, a thioether bridge or a lactam. Typically, cyclic derivatives containing a disulphide bond will contain two cysteines, which may be L-cysteine or D-cysteine. Advantageously, the N-terminal amino acid (e.g. R₁ in formula I) and the C-terminal amino acids are both cysteines. In such derivatives, as an alternative to cysteine, penicill amine (β,β-dimethyl-cysteine) can be used. Peptides containing thioether bridges are obtainable e.g. from starting compounds having a free cysteine residue at one end and a bromo-containing building block at the other end (e.g., bromo-acetic acid). Cyclisation can be carried out on solid phase by a selective deprotection of the side chain of cysteine. A cyclic lactam may be formed e.g. between the γ-carboxy group of glutamic acid and the ε-amino group of lysine. For example, cyclic lactams according to the invention have a Glu at the N-terminus (e.g. R₁ in formula I) and a Lys at the C-terminus. As an alternative to glutamic acid, it is possible to use aspartic acid. As an alternative to lysine, ornithine or diaminobutyric acid may be employed. Also, it is possible to make a lactam between the side chain of aspartic acid or glutamic acid at the C-terminus and the α-amino group of the N-terminal amino acid. This approach is extendable to β-amino acids (e.g., β-alanine). Alternatively, glutamine residues at the N-terminus or C-terminus can be tethered with an alkenedyl chain between the side chain nitrogen atoms (J.C. Phelan et al., J. of the American Chemical Society 119, 455 - 460 (1997)).
- Peptides of the invention, which are modified by substitution as defined in the claims. In the sequence of formula (I) one or more, preferably one or two, amino acids are replaced with another natural or unnatural amino acid as defined in the claims, e.g. with the respective D-analog, or a mimetic. The modifications are such that an α-helix conformation in the peptide is induced, increased or maintained. For example, in a peptide of formula (I), R₂, X₃ and/or X₄ may, independently from one another, be replaced by a α,α-disubstituted amino acid residue, α-aminoisobutyric acid, 1-amino-cyclopropane-1-carboxylic acid, 1-amino-cyclopentane-1-carboxylic acid, 1-amino-cyclohexane-1-carboxylic acid, 4-amino piperidine-4-carboxylic acid and 1-amino-cycloheptane-1-carboxylic acid.
- Peptides of the invention labeled with an enzyme, a fluorescent marker, a chemiluminescent marker, a metal chelate, paramagnetic particles, biotin, or the like. In such derivatives, the peptide of the invention is bound to the conjugation partner directly or by way of a spacer or linker group, e.g. a (peptidic) hydrophilic spacer. Advantageously, the peptide is attached at the N- or C-terminal amino acid. For example, biotin may be attached to the N-terminus of a peptide of the invention via a serine residue or the tetramer SerGlySerGly.
- Peptides of the invention carrying one or more protecting groups at a (potentially) reactive (side group), such as amino-protecting group, e.g. acetyl, or a carboxy-protecting group. For example, the C-terminal carboxy group of a compound of the invention may be present in form of a carboxamide function. Suitable protecting groups are commonly known in the art and further exemplified hereinbelow. Such groups may be introduced e.g. to enhance the stability of the compound against proteolytic degradation. If desired, such protecting groups are removed.
- Peptides of the invention fused or attached to another protein or peptide, e.g. a protein or peptide serving as internalization vector, such as another peptide facilitating cellular uptake, e.g. a "penetratin". An exemplary penetratin comprising derivative according to the invention is e.g. a peptide comprising the sixteen amino acid sequence from the homeodomain of the Antennapedia protein (D. Derossi et al., J. Biol. Chem. 269, 10444-10450 (1994)), particularly a peptide having the amino acid sequence: M-P-R-F-M-D-Y-WE-G-L-N-R-Q-I-K-I-W-F-Q-N-R-R-M-K-W-K-K, or comprising a peptide sequence disclosed by Y.-Z. Lin et al., J. Biol. Chem. 270,14255-14258 (1995)),
- Salts, especially acid addition salts, salts with bases or, where several salt-forming groups are present, mixed salts or internal salts. Exemplary salts are e.g. the salts described in the Examples. Preferred are pharmaceutically acceptable salts. However, it is also possible to use pharmaceutically unacceptable salts, e.g. for isolation or purification purposes.

Derivatives of a peptide of the invention also comprise fragments of such peptide as defined in the claims. Such fragments may be further derivatized as described in detail above.

More specifically, a preferred fragment according to the invention is an 8mer peptide, i.e. a peptide containing eight amino acid residues, of formula

F-X₂-R₂-R₃-W-X₃-X₄-R₄ (Ib),

wherein
R₂, R₃ and R₄, independently from one another, each have the meanings and preferences given for formula (I) above,
X₂ is methionine isoleucine, threonine, arginine, alanine or serine, preferably methionine;
X₃ is glutamic acid, threonine, alanine, phenylalanine or serine, preferably glutamic acid;
X₄ glycine, glutamine, threonine, alanine or aspartic acid, preferably glycine,
or a derivative as defined above of such fragment.

Also preferred is a fragment, which is a 9mer peptide having the formula

X₁-F-X₂-R₂-R₃-W-X₃-X₄-R₄ (Ic),

wherein
R₁, R₂, R₃ and R₄, independently from one another, each have the meanings and preferences given for formula (I) above,
X₁ is arginine, asparagine, alanine, threonine or valine; particularly arginine
X₂ is methionine, isoleucine, threonine, arginine, alanine or serine; preferably methionine;
X₃ is glutamic acid, threonine, alanine, phenylalanine or serine; preferably glutamic acid;
X₄ is glycine, glutamine, threonine, alanine or aspartic acid, preferably glycine,
or a derivative as defined above of such fragment.

Particularly preferred derivatives of peptide fragments of the invention contain the 8mer motif of formula (Ib) or the 9mer motif of formula (Ic) and also
- a suitable label means, e.g. an enzyme, a fluorescent marker, a chemiluminescent marker, a metal chelate, paramagnetic particles, biotin, or the like, and/or
- one or more protecting groups, e.g. as defined above, such as acetyl, and/or
- be fused or attached to another protein or peptide, e.g. a peptide as mentioned above.

Also included within the scope of the provided fragment derivatives are peptides of formula (Ib) or (Ic), wherein one or more, preferably one, two or three amino acid residues are replaced with another natural or unnatural amino as defined in the claims. The modifications are such that an α-helix conformation in the fragment is induced, increased or maintained. For example, in a peptide of formula (I), each of R₂, X₃ and/or X₄ may, independently from one another, be replaced by a α,α-disubstituted amino acid residue, such as α-aminoisobutyric acid (Aib), 1-amino-cyclopropane-1-carboxylic acid, 1-amino-cyclopentane-1-carboxylic acid, 1-amino-cyclohexane-1-carboxylic acid, 4-aminopiperidine-4-carboxylic acid, or 1-amino-cycloheptane-1-carboxylic acid. Such replacement may be combined with the above mentioned substitution by *ortho*-tyrosine. Also, in a 9mer fragment of formula (Ic), wherein R₂ is aspartic acid and the remaining variables have the meanings and preferences given above, X₁ may be replaced with NH₂-(CH₂)ₙ-CO-, wherein n is from 4 to 6, preferably a 6-amino-hexanoic acid residue. The N-terminal amino group of such fragment derivative will form a lactam with the side chain of aspartic acid.

Exemplary fragments include the following: P-A-F-T-H-Y-W-P, and, particularly, P-T-F-S-D-Y-W-P and P-R-F-M-D-Y-W-P, or derivatives thereof. Particularly preferred are fragments having the following amino acid sequences: R-F-M-D-Y-W-E-G-L and F-M-D-Y-W-E-G-L, or derivatives thereof.

Specially preferred derivatives of the invention are the derivatives used to exemplify the present invention, derivatives of the peptides above designated as being preferred, and derivatives of fragments as defined above.

A derivative according to the invention may involve one or multiple modifications as compared to a peptide of the invention, e.g. carry one or more of the above defined moieties. In other words, a derivative of the invention is intended to include compounds derivable from or based on a peptide of the invention or another derivative of the invention. The derivatives of the invention are capable of binding to MDM2 and of selectively inhibiting or blocking the binding of MDM2 to the p53 protein.

The compounds of the invention have useful, in particular pharmacologically useful properties. For example, they are useful in the treatment of diseases that respond to the inhibition of the p53-MDM2 interaction. As used hereinbefore or hereinbelow, the term "compound of the invention" includes peptides and derivatives of the peptides of the invention as defined in the claims.

The ability of a test compound to inhibit interaction between MDM2 and p53 can be shown by assays commonly known in the art, or modifications of known assays readily apparent to a person of ordinary skill in the art. Suitable assays include e.g. a binding assay determining binding of a test compound, e.g. a compound of the invention, to MDM2, an *in vitro* transcription assay or an assay as described in European Patent Application 95810576.9, corresponding to International Application No. PCT/EP 96/03957. Assays may be performed qualitatively or quantitatively and require comparison to one or more suitable controls.

A preferred binding assay is a competitive binding assay. The principle underlying a competitive binding assay is generally known in the art. Briefly, such binding assay is performed by allowing a compound to be tested for its capability to compete with a known, suitably labeled ligand, e.g. MDM2 or p53 for the binding site at a target molecule, e.g. p53 or MDM2 (depending on which molecule is used as known ligand). A suitably labeled ligand is e.g. a radioactively labeled ligand or a ligand which can be detected by its optical properties, such as absorbance or fluorescence. After removing unbound ligand and test compound the amount of labeled ligand bound to the target protein is measured. If the amount of bound ligand is reduced in the presence of the test compound, said compound is found to bind to the target molecule.

Further details of suitable assays are given in the Examples. For example, ELISA-type assays may be used wherein p53 or an appropriately labeled p53 peptide comprising the MDM2 binding site on p53 is immobilized and binding of MDM2 is competed for by a candidate inhibitor. Alternatively, MDM2 may be immobilized and binding of p53 is competed for by such candidate. Furthermore, an assay involving phage display of a candidate peptide, e.g. a phage ELISA assay, may be used.

The compounds of the invention are superior to the peptide having the amino acid sequence QETFSDLWKLLP corresponding to the correct p53 wild-type sequence in their ability to selectively inhibit the binding of p53 and MDM2.

The peptides and derivatives of the present invention can be readily prepared according to well-established, standard liquid or, preferably, solid-phase peptide synthesis methods, general descriptions of which are broadly available (see, for example, in J.M. Stewart and J.D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984); and Applied Biosystems 430A Users Manual, ABI Inc., Foster City, California), or they may be prepared in solution, by the liquid phase method or by any combination of solid-phase, liquid phase and solution chemistry, e.g. by first completing the respective peptide portion and then, if desired and appropriate, after removal of any protecting groups being present, by introduction of the residue X by reaction of the respective carbonic or sulfonic acid or a reactive derivative thereof.

Reactive derivatives of carbonic or sulfonic acids are preferably reactive esters, reactive anhydrides or reactive cyclic amides. Reactive carbonic acid or reactive sulfonic acid derivatives can also be formed in situ.

The reaction steps required e.g. for the synthesis of amide or sulfonamide bonds usually depend on the type of activation of the carboxylic or sulfo group participating in the reaction. The reactions normally run in the presence of a condensing agent or, when activating the carboxylic or sulfonic acids in the form of anhydrides, of an agent that binds the carboxylic or sulfonic acid formed. The reactions are especially carried out in a temperature range from -30 to +150 °C, preferably from +10 to +70 °C, and, most preferably, from +20 to +50 °C, if appropriate, in an inert gas atmosphere, e.g. under nitrogen or argon.

Synthesis proceeds in a stepwise, cyclical fashion by successively removing the NH₂ protecting group of the amino group to be reacted next and then coupling an activated fragment (e.g. an amino acid, di-, tri- or oligopeptide or a carboxylic acid or sulfonic acid, or a reactive derivative thereof, to the deprotected NH₂ (e.g. α- or β-NH₂). Preferably, activation of the COOH group of the amino acid to be reacted or the carboxyl or sulfo group of the acid to be attached by the condensation reaction is effected
(i) directly with a carbodiimide, with a carbonyl compound such as carbonyldiimidazole; with 1,2-oxazolium compounds; with acylamino compounds such as 2-ethoxy-1-ethoxycarbonyl-1,2-dihydroquinoline; with N-[(dimethylamino)-1H-1,2,3-triazolo[4,5-b]pyridin-1-ylmethylene]-N-methylmethanaminium hexafluorophosphate N-oxide (HATU) ; with an uronium compound such as 2-(1H-benzotriazol-1-yl)-1,1,3,3-tetramethyluronium tetrafluoroborate (HBTU); or phosphonium compounds such as benzotriazol-1-yl-oxy-tris(dimethylamino)-phosphonium hexafluoro phosphate (BOP) or benzotriazol-1-yl-oxy-pyrrolidino-phosphonium hexafluorophosphate (PyBOP);
(ii) via formation of the symmetric anhydride (obtainable, for example, by condensation of the corresponding acid in the presence of a carbodiimide or 1-diethylaminopropyne; symmetric anhydrides method), or an asymmetric anhydride, such as the respective carbonic or sulfonic acid bromide, chloride or fluoride, or
(iii) by formation of an "active ester", e.g. an amino- or amido ester, such as a 1-hydroxy-benzotriazole (HOBT) or N-hydroxysuccinimide ester, or an aryl ester, such as a penta-fluorophenyl, 4-nitrophenyl or 2,4,5-tetrachlorophenyl ester;
or by an appropriate combination of any of the reagents and reactions mentioned under (i) to (iii).

Useful acid binding agents that can be employed in the condensation reactions are, for example, alkaline metals, carbonates or bicarbonates, such as sodium or potassium carbonate or bicarbonate (if appropriate, together with a sulfate), or organic bases such as sterically hindered organic nitrogen bases, for example tri-lower alkylamines, such as N,N-diisopropyl-N-ethylamine, which can be used alone or in any appropriate combination.

Reactive groups in the monomers of ligands or in the resin-bound or free intermediates resulting from one or more coupling steps can be protected by third groups as protecting groups that are customarily used in peptide synthesis. Examples of protecting groups, their introduction and their removal are, for example, described in standard works such as "Protective groups in Organic Chemistry", Plenum Press, London, New York 1973; "Methoden der organischen Chemie", Houben-Weyl, 4. edition, Vol. 15/1, Georg-Thieme Verlag, Stuttgart 1974; Th. W. Greene, "Protective Groups in Organic Synthesis", John Wiley & Sons, New York 1981; Atherton et al., "Solid Phase Peptide Synthesis - A Practical Approach", IRL Press Oxford University, 1984; Jones, "The Chemical Synthesis of Peptides", Oxford Science Publications, Clavendon Press Oxford, 1991; and Bodanszky, "Peptide Chemistry", Springer Verlag Berlin, 1988. The term "protecting groups" comprises also resins used for solid phase synthesis, preferably those specifically mentioned above and below.

Examples for hydroxy protecting groups are acyl radicals, such as tert-lower alkoxycarbonyl radicals, for example tert-butoxycarbonyl, etherifying groups, such as tert-lower alkyl groups, for example t-butyl, or silyl- or tin radicals, such as tert-butyl-dimethylsilyl or the tri-n-butyltin radical.

Carboxy groups can be protected by groups as defined above for the C-terminal protecting groups Y, preferably by esterifying groups selected from those of the tert-butyl type, from benzyl, from trimethylsilylethyl and from 2-triphenylsilyl groups, or they can be protected as lower alkenyl esters, such as allylic esters.

Amino or guanidino (e.g. in H-Arg-OH) groups can be protected by removable acyl groups or by arylmethyl, etherified mercapto, 2-acyl-lower alk-1-enyl, a silyl group or an organic sulfonyl group or tin amino protecting groups; tert-butoxycarbonyl, allyloxycarbonyl, benzyl-oxycarbonyl, 4-nitrobenzyloxycarbonyl, 2-chlorobenzyloxycarbonyl, 2-bromobenzyloxy-carbonyl, diphenylmethoxycarbonyl, nitrophenylsulfenyl, 2,2,2-trichloroethoxycarbonyl, 2,2,5,7,8-pentamethylchroman-6-sulfonyl (PMC - very preferred), 2,2,4,6,7-pentamethyldihydrobenzofuran-5-sulfonyl (Pbf) or 4-methoxy-2,3,6-tri methylbenzenesulfonyl (Mtr) being especially preferred.

Carbamide groups (for example, in the side chains of asparagine and glutamine) can be protected at the nitrogen atom by arylmethyl groups, preferably triphenylmethyl (trityl) or analogues thereof with one or more lower alkoxy, such as methoxy, and/or lower alkyl, such as methyl, substituents in one or more phenyl rings.

Imino groups (e.g. in imidazole) can be protected by 2,4-dinitrophenyl, trityl, tert-butoxy-carbonyl or p-toluene sulfonyl, or (e.g. in indole) by formyl or tert-butoxycarbonyl.

Mercapto groups can be protected, e.g., by acetamidomethyl, by trityl or by p-methylbenzyl.

A large number of methods of removing protective groups in the final products or any inter-mediates are known in the art and comprise, inter alia, β-elimination, solvolysis, hydrolysis, alcoholysis, acidolysis, photolysis, enzymatical removal, treatment with a base or reduction.

The protective groups are usually removed after the complete synthesis of the resin-bound molecule by conventional methods of peptide chemistry, conveniently by treatment with 95 % trifluoroacetic acid (Fmoc-chemistry). In some cases, strong nucleophiles, such as dimethyl sulfide and/or 2-ethanedithiol, may be additionally added to capture the generated compounds resulting from the protecting groups, e.g. in a combination such as trimethyl-silyltrifluoro-methansulfonate/dimethylsulfide/trifluoroacetic acidlethanedithiol/m-cresol.

The two preferred methods of solid phase peptide synthesis are the Boc and the Fmoc methods, which are named with reference to their use of the tert-butoxycarbonyl (Boc) or 9-fluorenylmethyloxycarbonyl (Fmoc) group, respectively, to protect the α-NH₂ or α-NHR₃ of the amino acid residue to be coupled (see J. M. Stewart, J. D. Young, Solid-Phase Peptide Synthesis, 2n edn., Pierce, Rockford, Illinois (1984) or G. Barany, R.B. Merrifield, Solid-phase Peptide Synthesis, in: The Peptides, Vol. 2 (E. Gross, J. Meienhofer, eds.), Academic Press, New York (1979)); and E. Atherton and R.C. Sheppard, in Solid-Phase Peptide Synthesis-A Practical Approach, ed. D. Rickwood and B.D. Hames, IRL Press at Oxford University Press, Oxford, 1989), respectively).

Derivatives of the invention are prepared according to conventional methods involving de novo synthesis or starting from a peptide or another derivative of the invention.

In another aspect, the present invention provides means for use in a method for treating or preventing hyperproliferative disease by interfering with the interaction or binding between p53 and MDM2 in tumor cells. The method may comprise administering an effective amount of a compound of the invention to a warm blood animal, including a human, or tumor cells containing wild type p53. The administration of the compounds of the present invention may induce cell growth arrest or apoptosis. The present invention may be used to treat disease and or tumor cells comprising non-elevated MDM2 levels. Non elevated levels of MDM2 as used herein refers to MDM2 levels lower than those found in cells containing more than the normal copy number (2) of mdm2 or below about 10,000 molecules of MDM2 per cell as measured by ELISA and similar assays known in the art (Picksley et al., Oncogene 9, 2523-2529 (1994)).

Furthermore, the present invention relates to uses of a compound of the invention including its use in the purification of a binding partner, particularly MDM2; its use as a "lead compound" for drug development or design; its use in a method of identifying compounds which interfere with the binding of MDM2 to p53; its use in diagnosis, e.g. to measure the levels of MDM2 in blood samples in the case of leukemia or solid carcinomas, such as sarcomas or glioblastomas.

The invention relates also to pharmaceutical compositions comprising compounds of the invention, to their use in the therapeutic (including prophylactic) treatment of the hyperproliferative diseases and viral infections, to the compounds for said use and to the preparation of pharmaceutical preparations.

The pharmacologically acceptable compounds of the present invention may be used, for example, for the preparation of pharmaceutical compositions that comprise an effective amount of the active ingredient together or in admixture with a significant amount of inorganic or organic, solid or liquid, pharmaceutically acceptable carriers.

The invention provides a pharmaceutical composition that is suitable for admin istration to a warm-blooded animal, especially a human (or to cells or cell lines derived from a warm-blooded animal, especially a human, e.g. lymphocytes), for the treatment or prevention of (= prophylaxis against) a disease that responds to inhibition of the interaction of p53 with MDM2, comprising an amount of a peptide of the invention or a pharmaceutically acceptable derivative thereof, which is effective for said inhibition, together with at least one pharmaceutically acceptable carrier.

The pharmaceutical compositions according to the invention are those for enteral, such as nasal, rectal or oral, or parenteral, such as intramuscular or intravenous, administration to warm-blooded animals (humans and animals), that comprise an effective dose of the pharmacologically active ingredient, alone or together with a significant amount of a pharmaceutically acceptable carrier. The dose of the active ingredient depends on the species of warm-blooded animal, the body weight, the age and the individual condition, individual pharmacokinetic data, the disease to be treated and the mode of administration. The invention relates also to a method of treating diseases that respond to inhibition of the interaction of MDM2 and p53, which comprises administering a prophylactically or especially therapeutically effective amount of a compound according to the invention, especially to a warm-blooded animal, for example a human, that, on account of one of the mentioned diseases, requires such treatment. In a preferred embodiment the administered compound is a peptide or derivative of the invention.

The pharmaceutical compositions comprise from approximately 1 % to approximately 95%, preferably from approximately 20 % to approximately 90%, active ingredient. Pharmaceutical compositions according to the invention may be, for example, in unit dose form, such as in the form of ampoules, vials, suppositories, dragées, tablets or capsules.

The pharmaceutical compositions of the present invention are prepared in a manner known *per se,* for example by means of conventional dissolving, lyophilising, mixing, granulating or confectioning processes.

Solutions of the active ingredient, and also suspensions, and especially isotonic aqueous solutions or suspensions, are preferably used, it bei ng possible, for example in the case of lyophilized compositions that comprise the active ingredient alone or together with a carrier, for example mannitol, for such solutions or suspensions to be produced prior to use. The pharmaceutical compositions may be sterilized and/or may comprise excipients, for example preservatives, stabilisers, wetting and/or emulsifying agents, solubilisers, salts for regulating the osmotic pressure and/or buffers, and are prepared in a manner known *per se*, for example by means of conventional dissolving or lyophilising processes. The said solutions or suspensions may comprise viscosity-increasing substances, such as sodium carboxymethylcellulose, carboxymethylcellulose, dextran, poly vinylpyrrolidone or gelatin.

Suspensions in oil comprise as the oil component the vegetable, synthetic or semisynthetic oils customary for injection purposes. There may be mentioned as such especially liquid fatty acid esters that contain as the acid component a long-chained fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brasidic acid or linoleic acid, if desired with the addition of anti oxidants, for example vitamin E, β-carotene or 3,5-di-tert-butyl-4-hydroxytoluene. The alcohol component of those fatty acid esters has a maximum of 6 carbon atoms and is a mono- or poly-hydroxy, for example a mono-, di- or tri-hydroxy, alcohol, for example methanol, ethanol, propanol, butanol or pentanol or the isomers thereof, but especially glycol and glycerol. The following examples of fatty acid esters are there fore to be mentioned: ethyl oleate, isopropyl myristate, isopropyl palmitate, "Labrafil M 2375" (poly oxyethylene glycerol trioleate, Gattefossé, Paris), "Miglyol 812" (triglyceride of saturated fatty acids with a chain length of C₈ to C₁₂, Hüls AG, Germany), but especially vegetable oils, such as cottonseed oil, almond oil, olive oil, castor oil, sesame oil, soybean oil and more especially groundnut oil.

The injection compositions are prepared in customary manner under sterile conditions; the same applies also to introducing the compositions into ampoules or vials and sealing the containers.

Pharmaceutical compositions for oral administration can be obtained by combining the active ingredient with solid carriers, if desired granulating a resulting mixture, and processing the mixture, if desired or necessary, after the addition of appropriate excipients, into tablets, dragée cores or capsules. It is also possible for them to be incorporated into plastics carriers that allow the active ingredients to diffuse or-be released in measured amounts.

Suitable carriers are especially fillers, such as sugars, for example lactose, saccharose, mannitol or sorbitol, cellulose preparations and/or calcium phosphates, for example tricalcium phosphate or calcium hydrogen phosphate, and binders, such as starch pastes using for example corn, wheat, rice or potato starch, gelatin, tragacanth, methylcellulose, hydroxypropylmethylcellulose, sodium carboxymethylcellulose and/or poly vinylpyrrolidone, and/or, if desired, disintegrates, such as the above- mentioned starches, also carboxymethyl starch, crosslinked polyvinylpyrrolidone, agar, alginic acid or a salt thereof, such as sodium alginate. Excipients are especially flow conditioners and lubricants, for example silicic acid, talc, stearic acid or salts thereof, such as magnesium or calcium stearate, and/or polyethylene glycol. Dragée cores are provided with suitable, optionally enteric, coatings, there being used, *inter alia,* concentrated sugar solutions which may comprise gum arabic, talc, polyvinylpyrrolidone, polyethylene glycol and/or titanium dioxide, or coating solutions in suitable organic solvents, or, for the preparation of enteric coatings, solutions of suitable cellulose preparations, such as ethylcellulose phthalate or hydroxypropylmethylcellulose phthalate. Capsules are dry-filled capsules made of gelatin and soft sealed capsules made of gelatin and a plasticiser, such as glycerol or sorbitol. The dry-filled capsules may comprise the active ingredient in the form of granules, for example with fillers, such as lactose, binders, such as starches, and/or glidants, such as talc or magnesium stearate, and if desired with stabilisers. In soft capsules the active ingredient is preferably dissolved or suspended in suitable oily excipients, such as fatty oils, paraffin oil or liquid polyethylene glycols, it being possible also for stabilisers and/or antibacterial agents to be added. Dyes or pigments may be added to the tablets or dragée coatings or the capsule casings, for example for identification purposes or to indicate different doses of active ingredient.
The following Examples serve to illustrate the present invention, but should not be construed as a limitation thereof. The invention particularly relates to the specific embodiments (e.g. peptides, methods for their preparation, and assays as described in these Examples.
Abbreviations: Acrld = thioether resulting from the reaction of a Cys-sulfhydryl group in the peptide with 6-acryloyl-2-(dimethylamino)naphtalene; o/n = overnight; Aib: α-aminoisobutyric acid; Ac₃c: 1-amino-cyclopropane-1-carboxylic acid.

### Examples

### Example 1: Synthesis of N-acylated peptide derivatives

The below-identified peptides are synthesised on a Milligen 9050 automated peptide synthesizer (continuous flow; Millipore, Bedford, MA, USA), starting with an Fmoc-PAL-PEG-PS resin (see Albericio, F. et al, J. Org. Chem., 55 (1990) 3730-3743) for establishing the C-terminal carboxamide, and using chemical protocols based on the fluorenylmethoxycarbonyl chemistry (see E. Atherton and R.C. Sheppard, in Solid-Phase Peptide Synthesis-A Practical Approach, eds: R. Rickwood and B.D. Hames, IRL Press at Oxford University Press, Oxford, 1989). The required Fmoc-amino acids (3 equivalents) are incorporated using their 2,4,5-trichlorophenyl esters (single coupling) with minimum reaction times of 30 min (see 9050 Plus PepSynthesizer User's Guide, Millipore Corporation, Bedford, MA, 1992). Side chains are protected with the following groups:
*tert*-butyl for aspartic acid, glutamic acid, tyrosine, serine and threonine;
*tert*-butyloxycarbonyl for lysine and tryptophan;
2,2,5,7,8-pentamethyl-chroman-6-sulfonyl for arginine;
trityl for histidine, cysteine, asparagine, and glutamine.

The complete peptide resins obtained after the final coupling reaction are simultaneously deprotected and cleaved by treatment with trifluoroacetic acid/water/ethanedithiol (76:4:20, v/v/v) for 3 h at room temperature. The complete peptide resins obtained after the final coupling reaction are simultaneously deprotected and cleaved by treatment with trifluoroacetic acid/water/ethanedithiol (76:4:20, v/v/v) for 3 h at room temperature. The filtrate from each cleavage reaction is precipitated in diisopropyl ether-petroleum ether (1:1, v/v) at 0° C, and the precipitates are collected by filtration. The crude compounds are dissolved in 2N AcOH/acetonitrile (1:1, v/v) to remove the Nⁱⁿ-carboxy group from the side chain of tryptophan. The course of the reactions is monitored by analytical reversed-phase HPLC. After 2 h at 40°C, the solutions are concentrated to dryness and the crude peptides are purified by reversed-phase medium-pressure liquid chromatography using a C₁₈ column eluted with an acetonitrile-water gradient containing 0.1% trifluoroacetic acid (Merck LICHROPREP RP-18, 15-25 µm bead diameter, reversed phase column material based on C₁₈-derivatised silicagel, Merck, Darmstadt, FRG; column length 46 cm, diameter 3.6 cm; flow rate 53.3 ml/min; detection at 215 nm). Mass spectrometric analyses (matrix-assisted laser-desorption ionization time-of-flight mass spectrometry, MALDI-TOF) reveal molecular masses within 0.1% of the expected values (positive or negative ion mode). Quantitative amino acid analyses of the final products reveal amino acid compositions within 5% of the expected values. The purity of the peptides is verified by reversed-phase analytical HPLC on a Nucleosil column (250 x 4.0 mm; 5 mm, 100): linear gradient over 10 min of MeCN/0.09% TFA and H₂O/0.1 % TFA from 1:49 to 3:2; flow rate 2.0 ml/min, detection at 215 nm (HPLC System A); HPLC System B: linear gradient over 10 min of MeCN/0.09% TFA and H₂O/0.1% TFA from 1:49 to 1:0; flow rate 2.0 ml/min, detection at 215 nm.

The peptides are as follows:
- Ac-Thr-Gly-Pro-Ala-Phe-Thr-His-Tyr-Trp-Ala-Thr-Phe-NH₂ (TFA salt);
   Mass spectral analysis (negative-ion mode): 1441.7 (calc. 1441.6, C₇₁H₉₂N₁₆O₁₇), t_{R}^{(retention time)} = 8.08 min (HPLC System A).
- Ac-Met-Pro-Arg-Phe-Met-Asp-Tyr-Trp-Glu-Gly-Leu-Asn-NH₂ (TFA salt);
   Mass spectral analysis (negative-ion mode): 1598.9 (calc. 1598.9, C₇₃H₁₀₁N₁₈O₁₉S₂), t_{R}= 8.82 min (HPLC System A).
- Ac-Gln-Pro-Thr-Phe-Ser-Asp-Tyr-Trp-Lys-Leu-Leu-Pro-NH₂ (TFA salt);
   Mass spectral analysis (negative-ion mode): 1534.8 (calc. 1534.8, C₇₅H₁₀₅N₁₆O₁₉), t_{R}= 8.73 min (HPLC System A).
- Ac-Pro-Ala-Phe-Thr-His-Tyr-Trp-Pro-NH₂ (TFA salt);
   Mass spectral analysis (negative-ion mode): 1060.3 (calc. 1060.2, C₅₄H₆₇N₁₂O₁₁), t_{R}= 8.21 min (HPLC System A).
- Ac-Pro-Thr-Phe-Ser-Asp-Tyr-Trp-Pro-NH₂
   Mass spectral analysis (negative-ion mode): 1052.0 (calc. 1052.1, C₅₂H₆₃N₁₀O₁₄), t_{R}= 7.97 (HPLC System A).
- Ac-Pro-Arg-Phe-Met-Asp-Tyr-Trp-Pro-NH₂ (TFA salt);
   Mass spectral analysis (negative-ion mode): 1151.6 (calc. 1151.3, C₅₆H₇₂N₁₃O₁₂S₁), t_{R}= 8.42 (HPLC System A).
- Ac-Gln-Glu-Thr-Phe-Ser-Asp-Leu-Trp-Lys-Leu-Leu-Pro-NH₂ (TFA salt) (wild-type sequence)
   Mass spectral analysis (negative-ion mode): 1517.1 (calc. 1516.8, C₇₂H₁₀₇N₁₆O₂₀), t_{R}= 9.30 (HPLC System A), t_{R}= 6.65 (HPLC System B)
- Ac-Gln-Pro-Thr-Phe-Ser-Asp-Leu-Trp-Lys-Leu-Leu-Pro-NH₂ (TFA salt)
   Mass spectral analysis (negative-ion mode): 1485.0 (calc. 1484.8, C₇₂H₁₀₇N₁₆O₁₈), t_{R}= 9.32 (HPLC System A), t_{R}= 6.66 (HPLC System B)
- Ac-Gln-Gtu-Thr-Phe-Ser-Asp-Tyr-Trp-Lys-Leu-Leu-Pro-NH₂ (TFA salt)
   Mass spectral analysis (negative-ion mode): 1567.3 (calc. 1566.8, C₇₅H₁₀₅N₁₆O₂₁), t_{R}= 8.55 (HPLC System A), t_{R}= 6.19 (HPLC System B).
- Ac-Val-Gln-Asn-Phe-Ile-Asp-Tyr-Trp-Thr-Gln-Gln-Phe-NH₂
   Mass spectral analysis (negative-ion mode): 1628.8 (calc. 1628.8, C₇₈H₁₀₃N₁₈O₂₁), t_{R}= 7.03 (HPLC System A);
- Ac-Ile-Asp-Arg-Ala-Pro-Thr-Phe-Arg-Asp-His-Trp-Phe-Ala-Leu-Val-NH₂ (TFA salt)
   Mass spectral analysis (negative-ion mode): 1883.9 (calc. 1884.2, C₈₉H₁₂₈N₂₅O₂₁), t_{R}= 8.57 min (HPLC System A)
- Ac-Pro-Arg-Pro-Ala-Leu-Val-Phe-Ala-Asp-Tyr-Trp-Glu-Thr-Leu-Tyr-NH₂ (TFA salt)
   Mass spectral analysis (negative-ion mode): 1881.6 (calc. 1881.2, C₉₂H₁₂₇N₂₀O₂₃), t_{R}= 9.58 min (HPLC System A); t_{R}= 6.88 min (HPLC System B).
- Ac-Pro-Ala-Phe-Ser-Arg-Phe-Trp-Ser-Asp-Leu-Ser-Ala-Gly-Ala-His-NH₂ (TFA salt)
   Title compound: Mass spectral analysis (negative-ion mode): 1688.6 (calc. 1688.9, C₇₈H₁₀₇N₂₂O₂₁), t_{R}= 9.09 min (HPLC System A); t_{R}= 6.48 min (HPLC System B)

If desired, the peptide derivatives contain a free N-terminal amino group. Peptides with a free N-terminal amino group include:
- H-Thr-Gly-Pro-Ala-Phe-Thr-His-Tyr-Trp-Ala-Thr-Phe-NH₂ (TFA salt)
   Mass spectral analysis (negative-ion mode): 1396.6 (calc. 1396.6, C₆₉H₈₇N₁₆O₁₆), t_{R}= 7.86 min (HPLC System A).
- H-Met-Pro-Arg-Phe-Met-Asp-Tyr-Trp-Glu-Gly-Leu-Asn-NH₂ (TFA salt)
   Mass spectral analysis (negative-ion mode): 1556.6 (calc. 1556.8, C₇₁H₉₉N₁₈O₁₈S₂), t_{R}= 7.92 min (HPLC System A).

### Example 2: Synthesis of Cys(Acrld) peptide derivatives

I. Ac-Cys(Acrld)-Gly-Gln-Pro-Thr-Phe-Ser-Asp-Tyr-Trp-Lys-Leu-Leu-Pro-NH₂ (TFA salt) Ac-Cys-Gly-Gln-Pro-Thr-Phe-Ser-Asp-Tyr-Trp-Lys-Leu-Leu-Pro-NH₂ (TFA salt) is obtained analogously to Example 1 (Mass spectral analysis (negative-ion mode): 1694.7 (calc. 1695.0, C₈₀H₁₁₃N₁₈O₂₁S₁), t_{R}= 8.39 (HPLC System A)).
   To a solution of Ac-Cys-Gly-Gln-Pro-Thr-Phe-Ser-Asp-Tyr-Trp-Lys-Leu-Leu-Pro-NH₂ (18 µmol) in 20 ml of degassed phosphate buffer (pH= 7.5) is added 6-acryloyl-2-(dimethylamino)naphtalene (2-fold excess; Molecular Probes, Inc., Leiden, The Netherlands) dissolved in 2 ml of acetonitrile. The solution is stirred overnight at room temperature under an argon atmosphere. After completion of the reaction, 1 ml of trifluoroacetic acid is added and the solution is concentrated to dryness. The compound is purified by reversed-phase medium-pressure liquid chromatography.
   Title compound: Mass spectral analysis (negative-ion mode): 1920.4 (calc. 1920.3, C₉₅H₁₂₈N₁₉O₂₂S₁), t_{R}= 9.20 (HPLC System A); t_{R}= 6.60 (HPLC System B).
II. Ac-Cys(Acrd)-Gly-Pro-Thr-Phe-Ser-Asp-Leu-Trp-Pro-NH₂ (TFA salt) Ac-Cys-Gly-Pro-Thr-Phe-Ser-Asp-Leu-Trp-Pro-NH₂ is obtained analogously to Example 1 (Mass spectral analysis (negative-ion mode): 1162.0 (calc. 1162.3, C₅₄H₇₃N₁₂O₁₅S₁), t_{R}= 8.00).
   The title compound (II) is obtained analogously to the previous example (I).
   Title compound: Mass spectral analysis (negative-ion mode): 1387.6 (calc. 1387.6, C₆₉H₈₈N₁₃O₁₆S₁), t_{R}= 9.63 (HPLC System A), t_{R}= 6.87 (HPLC System B).
III. Ac-Cys(Acrd)-Pro-Thr-Phe-Ser-Asp-Leu-Trp-Pro-NH₂ (TFA salt) Ac-Cys-Pro-Thr-Phe-Ser-Asp-Leu-Trp-Pro-NH₂ is obtained analogously to Example 1. (Mass spectral analysis (negative-ion mode): 1105.5 (calc. 1105.3, C₅₂H₇₀N₁₁O₁₄S₁), t_{R}= 8.25 (HPLC System A).
   The title compound is obtained analogously to the above example.
   Title compound: Mass spectral analysis (negative-ion mode): 1330.6 (calc. 1330.6, C₆₇H₈₅N₁₂O₁₅S₁), t_{R}= 9.82 (HPLC System A); t_{R}= 7.02 (HPLC System B).

### Example 3: Synthesis of biotinylated peptide derivatives

- Biotin-Ser-Gly-Ser-Gly-Gln-Glu-Thr-Phe-Ser-Asp-Leu-Trp-Lys-Leu-Leu-Pro-NH2 (TFA salt) (wild-type sequence)
   (+)-Biotin (3 equivalents; Fluka, Buchs, Switzerland) is coupled with N-[(dimethylamino)1H-1,2,3-triazolo[4,5-b]pyridin-1-ylmethylene]-N-methylmethan-aminium hexafluorophosphate N-oxide (3 equiv.; double coupling; PerSeptive Biosystems, Hamburg, Germany) in the presence of diisopropylethylamine (6 equiv.)
   Mass spectral analysis (negative-ion mode): 1989.5 (calc. 1989.3, C₉₀H₁₃₅N₂₂O₂₇S₁), t_{R}= 9.02 (HPLC System A), t_{R}= 6.55 (HPLC System B)
- Biotin-Ser-Gly-Ser-Gly-Gin-Pro-Thr-Phe-Ser-Asp-Leu-Trp-Lys-Leu-Leu-Pro-NH₂ (TFA salt).
   Mass spectral analysis (negative-ion mode): 1957.9 (calc. 1957.3, C₉₀H₁₃₅N₂₂O₂₅S₁), t_{R}= 9.04 (HPLC System A), t_{R}= 6.57 (HPLC System B).
- Biotin-Ser-Gly-Ser-Gly-Gln-Glu-Thr-Phe-Ser-Asp-Tyr-Trp-Lys-Leu-Leu-Pro-NH₂ (TFA salt).
   Mass spectral analysis (negative-ion mode): 2039.3 (calc. 2039.3, C₉₃H₁₃₃N₂₂O₂₈S₁), t_{R}= 8.46 (HPLC System A).
- Biotin-Ser-Met-Pro-Arg-Phe-Met-Asp-Tyr-Trp-Glu-Gly-Leu-Asn-Arg-Gln-lie-Lys-lie-Trp-Phe-Gln-Asn-Arg-Arg-Met-Lys-Trp-Lys-Lys-NH₂ (TFA salt)
   Mass spectral analysis (negative-ion mode): 4098.4 (calc. 4099.0, C₁₈₈H₂₈₄N₅₅O₄₁ S₄), t_{R}= 9.08 min (HPLC System A); t_{R}= 6.41 min (System B). This derivative comprises a biotin label, serine as spacer, a peptide of the invention and the penetratin sequence Arg-Gln-Ile-Lys-Ile-Trp-Phe-Gln-Asn-Arg-Arg-Met-Lys-Trp-Lys-Lys from the homeodomain of the Antennapedia protein (D. Derossi, J. Biol. Chem. 269, 10444-10450 (1994)).
- Ac-Ala-Ala-Val-Ala-Leu-Leu-Pro-Ala-Val-Leu-Leu-Ala-Leu-Leu-Ala-Pro- βAla-Met-Pro-Arg-Phe-Met-Asp-Tyr-Trp-Glu-Gly-Leu-Asn-βAla-Lys(Biotin)-NH₂ (TFA salt) The peptide contains the internalization vector: Ala-Ala-Val-Ala-Leu-Leu-Pro-Ala-Val-Leu-Leu-Ala-Leu-Leu-Ala-Pro (Lin et al., J. Biol. Chem. 270, 14255-14258 (1995)).

The peptide is synthesised as described in Example 1 using N^{a}-Fmoc-Lys(Aloc)-OH. After the incorporation of the last residue, the side chain of lysine is selectively deprotected with tetrakis(triphenylphosphine) palladium (0) in the presence of trimethylsilylacetate and 4-(trimethylsilyl)morpholine dissolved in dichloromethane. The deprotection is carried out in an argon atmosphere for 2 h at room temperature, followed by washing with dichloromethane (4 x 1 min), *N*-methylpyrrolidin-2-one (4 x 1 min), 0.05 M sodium diethyldithiocarbamate in DMF containing 0.5% of diisopropylethylamine (4 x 1 min), and *N*-methylpyrrolidin-2-one (4 x 1 min). The incorporation of (+)-biotin to the side chain of lysine is mediated by *N-*[dimethylamino)1*H*-1,2,3-triazolo[4,5-*b*)pyridin-1-ylmethylene]-*N*-methyimethan-aminium hexafluorophosphate *N*-oxide in the presence of diisopropyl ethylamine.
Title compound: Mass spectral analysis (negative-ion mode): 3593.7 (calc. 3593.4, C₁₆₉H₂₆₅N₄₀O₄₀S₃), t_{R}= 9.15 (HPLC System B).

### Example 4: Cyclic peptide derivatives containing disulphide bond

Cyclic peptides containing a disulphide bond are synthesized from the respective cysteinyl peptides as follows: the cysteinyl peptide (20 mg; in the following referred to as starting compound) is dissolved in a 0.1 M solution of ammonium bicarbonate (20 ml). The mixture is left to stand open to atmosphere. Aliquots of the solution are removed at different times and analysed by analytical HPLC. After 24 h, the reaction mixture is concentrated to dryness. The crude compound is dissolved in water and injected directly in a medium-pressure liquid chromatography system as described above, and the title compound is obtained.

Ac-Cys-Thr-Phe-Ser-Asp-Tyr-Trp-Cys-NH₂ is obtained analogously to Example 1.
Starting compound: Mass spectral analysis (negative-ion mode): 1064.6 (calc. 1064.2, C₄₈H₅₉N₁₀O₁₄S₂), t_{R}= 8.15 (HPLC System A).
- Ac-Cys-Thr-Phe-Ser-Asp-Tyr-Trp-Cys-NH₂

   S---------------------------------------S

   Title compound: Mass spectral analysis (negative-ion mode): 1062.2 (calc. 1062.2, C₄₈H₅₇N₁₀O₁₄S₂), t_{R}= 7.96 (HPLC System A).
   Ac-Cys-Ala-Phe-Thr-His-Tyr-Trp-Cys-NH₂ (TFA salt);
   Starting compound: Mass spectral analysis (negative-ion mode): 1070.0 (calc. 1070.2, C₅₀H₆₁N₁₂O₁₁S₂), t_{R}= 8.35 (HPLC System A).
- Ac-Cys-Ala-Phe-Thr-His-Tyr-Trp-Cys-NH₂ (TFA salt)

   S---------------------------------------S

   Mass spectral analysis (positive-ion mode): 1070.4 (calc. 1070.2, C₅₀H₆₁ N₁₂O₁₁ S₂), t_{R}= 8.13 min (HPLC System A).
   Ac-Cys-Arg-Phe-Met-Asp-Tyr-Trp-Cys-NH₂ (TFA salt)
   Starting compound: Mass spectral analysis (negative-ion mode): 1163.7 (calc. 1163.4, C₅₂H₆₈N₁₃O₁₂S₃), t_{R}= 8.67 (HPLC System A).
- Ac-Cys-Arg-Phe-Met-Asp-Tyr-Trp-Cys-NH₂ (TFA salt)

   S---------------------------------------S

   Mass spectral analysis (negative-ion mode): 1161.1 (calc. 1161.4, C₅₂H₆₆N₁₃O₁₂S₃), t_{R}= 8.33 min (HPLC System A).

As an alternative to cysteine, penicillamine (β,β-dimethyl-cysteine) can be used. Also, L-cysteine may be changed for D-cysteine either at the N- or C- terminus, or in both sides. Peptides containing thioether bridges are formed from starting compounds having a free cysteine residue at the C-terminus and a bromo-containing building block at the N-terminus (e.g., bromo-acetic acid). Cyclisation can be carried out on solid phase by a selective deprotection of the side chain of cysteine (Mayer, J.P. et al., Tetrahedron Lett. 361411, 7387-7390 (1995)).

### Example 5: Synthesis of lactam peptide derivatives

The peptide is synthesised manually on a 4-(2',4'-dimethoxyphenyl-aminomethyl)-phenoxy-resin (Novabiochem, Laufelfingen, Switzerland), employing the fluorenylmethoxycarbonyl strategy. Fmoc-removal is with piperidine/dimethylacetamide (1:4, v/v; 6 x 2 min), followed by washing with methanol (3 x 1 min), *N*-methylpyrrolidin-2-one (2 x 1 min), methanol (3 x 1 min), and *N*-methylpyrrolidin-2-one (3 x 2 min). Amino acid side chains are protected with the following groups: *tert*-butyl for threonine, serine, aspartic acid and tyrosine; 2,2,5,7,8-pentamethyl-chroman-6-sulfonyl for arginine; tert-butyloxycarbonyl for tryptophan; allyl for glutamic acid; and allyloxycarbonyl for lysine. The required Fmoc-derivatives of tryptophan, tyrosine, threonine, serine, aspartic acid, arginine, methionine, phenylalanine and alanine are incorporated using their 2,4,5-trichlorophenyl esters (2 equiv.) in the presence of 1-hydroxybenzotriazole (2 equiv.) and diisopropylethylamine (0.75 equiv.). The incorporation of N^{α}-Fmoc-Lys(Aloc)-OH (2 equiv.; PerSeptive Biosystems, Hamburg, Germany) and N^{α}-Fmoc-Glu(OAll)-OH (2 equiv.; Millipore, Bedford, MA, U.S.A.) is accomplished with benzotriazole-1-yl-oxy-tris-(dimethylamino)-phosphonium hexafluorophosphate/1-hydroxybenzotriazole (1:1;2 equiv.) in the presence of diisopropylethylamine (4 equiv.) Coupling is achieved by first dissolving the Fmoc-amino acid, diisopropylethylamine, and the coupling reagent in *N*-methylpyrrolidin-2-one, then waiting 3 min for preactivation, adding the mixture to the resin, and finally shaking for at least 45 min. After the incorporation of the last residue, the side chains of glutamic acid and lysine are selectively deprotected with tetrakis(triphenylphosphine) palladium (0) (Fluka, Buchs, Switzerland) in the presence of trimethylsilylacetate and 4-(trimethylsilyl)morpholine dissolved in dichloromethane. The deprotection is carried out in an argon atmosphere for 2h at room temperature, followed by washing with dichloromethane (4 x 1 min), *N*-methylpyrrolidin-2-one (4 x 1 min), 0.05 M sodium diethyldithiocarbamate in DMF containing 0.5% of diisopropylethylamine (4 x 1 min), and *N*-methylpyrrolidin-2-one (4 x 1 min). Intramolecular cyclisation on the solid support is accomplished with benzotriazoie-1-yl-oxy-tris-(dimethylamino)-phosphoniumhexafluorophosphate/1-hydroxybenzotriazole (1:1; 6 equiv.; double coupling) in the presence of diisopropylethylamine (12 equiv.). The complete peptide resin obtained after the cyclisation step is simultaneously deprotected and cleaved by treatment with trifluoroacetic acid/water/ethanedithiol (76:4:20, v/v/v) for 3 h at room temperature. The filtrate is precipitated in diisopropyl ether-petroleum ether (1:1, v/v) at 0° C, and the precipitate is collected by filtration. The crude compound is dissolved in 2N AcOH/acetonitrile (1:1, v/v) to remove the Nⁱⁿ-carboxy group from the side chain of tryptophan. The courses of the reactions are monitored by analytical reversed-phase HPLC. After 2 h at 40 °C, the solution is concentrated to dryness and the crude peptide is purified by medium-pressure liquid chromatography as described above.
- Ac-Glu-Thr-Phe-Ser-Asp-Tyr-Trp-Lys-NH₂ (TFA salt)

   ---------------CO-NH---------------

   Mass spectral analysis (negative-ion mode): 1097.5 (calc. 1097.2. C₅₃H₆₆N₁₁O₁₅), t_{R}= 7.49 min (HPLC System A).
- Ac-Glu-Arg-Phe-Met-Asp-Tyr-Trp-Lys-NH₂ (TFA salt)

   ---------------CO-NH---------------

   Mass spectral analysis (negative-ion mode): 1196.7 (calc. 1196.4, C₅₇H₇₅N₁₄O₁₃S₁), t_{R}= 8.09 min (HPLC System A).

As an alternative to glutamic acid, it is possible to use aspartic acid. As an alternative to lysine, ornithine or diaminobutyric acid may be used. As an alternative to side-side cyclisation, it is possible to make a lactam between the side chain of aspartic acid or glutamic acid at the C-terminus and the α-amino group of the N-terminal amino acid. This approach can also be expanded to β-amino acids (e.g., β-alanine).

The following peptides are synthesised as described in Example 1:
- Ac-Phe-Met-Aib-Tyr-Trp-Aib-Gly-Leu-NH₂
   Title compound: Mass spectral analysis (negative-ion mode): 1026.5 (calc. 1026.3, C₅₂H₆₉N₁₀O₁₀S₁), t_{R}= 7.81 (HPLC System B).
- Ac-Arg-Phe-Met-Aib-Tyr-Trp-Aib-Gly-Leu-NH₂
   Title compound: Mass spectral analysis (negative-ion mode): 1182.6 (calc. 11182.4, C58H81 N₁₄O₁₁ S₁), t_{R}= 7.09 (HPLC System B).
- Ac-Arg-Phe-Met-Aib-Tyr-Trp-Glu-Ac₃c-Leu-NH₂ (TFA salt)
   Title compound: Mass spectral analysis (negative-ion mode): 1252.7 (calc. 1252.5, C61 H₈₃N₁₄O₁₃S₁), t_{R}= 6.91 (HPLC System B).
- Ac-Phe-Met-Aib-Tyr-Trp-Aib-Ac₃c-Leu-NH₂
   Title compound: Mass spectral analysis (negative-ion mode): 1052.3 (calc. 1052.3, C54H71 N₁₀O₁ S₁), t_{R}= 8.03 (HPLC System B).
- Ac-Phe-Met-Aib-Tyr-Trp-Glu-Ac₃c-Leu-NH₂
   The peptide is synthesised as described in Example 5. The incorporation of N^{a}-Fmoc-1-amino-cyclopropane-1-carboxylic acid (2 equiv.) is carried out with benzotriazole-1-yl-oxy-tris-(dimethylamino)-phosphonium hexafluorophosphate/N-hydroxybenzotriazole (1:1; 2 equiv.) in the presence of diisopropylethylamine (5 equiv.). N^{a}-Fmoc-aminoisobutyric acid (2 equiv.) is coupled with benzotriazole-1-yl-oxy-tris-(dimethylamino)-phosphonium hexafluorophosphate/N-hydroxybenzotriazole (1:1; 2 equiv.; first coupling) and N-[(dimethylamino)-1H-1,2,3-triazolo-[4,5-b]pyridin-1-ytmethylene]-N-methylmethanaminium hexafluorophosphate N-oxide (2 equiv.; second coupling) in the presence of diisopropyl ethylamine (5 equiv.). A second coupling for glutamic acid and methionine (2 equiv.) is performed with N-[(dimethylamino)-1 H-1,2,3-triazolo-[4,5-b]pyridin-1-ylmethylene]-N-methylmethanaminium hexafluorophosphate N-oxide (2 equiv.; second coupling) in the presence of diisopropylethylamine (5 equiv.). The complete peptide resin obtained after the final coupling reaction is simultaneously deprotected and cleaved by treatment with trifluoro acetic acid/H₂O (95:5, v/v) for 3 h at room temperature. The filtrate from the cleavage reaction is precipitated in diisopropyl ether/petroleum ether (1:1, v/v, 0 °C), and the precipitate collected by filtration. The crude peptide is purified as described in Example 1.
   Title compound: Mass spectral analysis (negative-ion mode): 1096.4 (calc. 1096.3, C₅₅H₇₁ N₁₀O₁₂S1), t_{R}= 7.62 (HPLC System B).

The starting material is prepared as follows:
a) N^{a}-Fmoc-1-amino-cyclopropane-1-carboxylic acid
   The title compound is synthesised starting from 1-amino-cyclopropane-1-carboxyfic acid (Fluka, Buchs, Switzerland) according to a procedure known in the art (see E. Atherton et al., in: Solid-Phase Peptide Synthesis - A Practical Approach; D. Rickwood and B.D. Hames, IRL Press at Oxford University Press, Oxford, 1989): R_{f} = 0.44 (chloroform:methanol:water:acetic acid = 850:130:15:5, v/v/v/v). m.p. = 223 - 225 °C.

### Example 6: Synthesis of peptide fragment derivatives

The below identified peptide fragment derivatives are synthesised analogously to the method described in Example 1 above:
- Ac-Arg-Phe-Met-Asp-Tyr-Trp-Glu-Gly-Leu-NH₂ (TFA salt)
   Mass spectral analysis (negative-ion mode): 1256.4 (calc. 1256.4, C₅₉H₇₉N₁₄O₁₅S₁), t_{R}= 8.69 (HPLC System A), t_{R}= 7.02 (HPLC System B).
- Ac-Phe-Met-Asp-Tyr-Trp-Glu-Gly-Leu-NH₂
   Mass spectral analysis (negative-ion mode): 1100.5 (calc. 1100.3, C₅₃H₆₇N₁₀O₁₄S₁), t_{R}= 9.38 (HPLC System A), t_{R}= 6.76 (HPLC System B).
- Ac-Phe-Met-Aib-Tyr-Trp-Glu-Gly-Leu-NH₂
   Mass spectral analysis (negative-ion mode): 1070.4 (calc. 1070.3, C₅₃H₆₉N₁₀O₁₂S₁), t_{R}= 7.14 (HPLC System B).
- Ac-Phe-Met-Asp-Tyr-Trp-Aib-Gly-Leu-NH₂
   Mass spectral analysis (negative-ion mode): 1056.2 (calc. 1056.2, C₅₂H₆₇N₁₀O₁₂S₁), t_{R}= 7.07 (HPLC System B).

### Example 7: Fluorescence assay

The DNA region of the mdm2 gene encoding the first 188 amino acids of the protein is obtained by Polymerase Chain Reaction (PCR) amplification of the mdm2 gene. The oligonucleotides used for PCR are designed such that a BamHI restriction site is introduced at the 5' extremity of the gene and an EcoRI restriction site at its 3' end. The PCR fragments digested by BamHI and EcoRI are ligated with a BamHI / EcoRI cleaved pGEX-2T vector. The resulting vector comprises a fusion gene consisting of the full length sequence of glutathione-S-transferase of S. japonicum, a linker sequence, and the N-terminal 188 amino acids of HDM2, in the 5' to 3' order. The complete gene is sequenced on both strands and the recombinant plasmid is introduced into *E. coli* strain BL21 (Novagen).
Glutathione-S-transferase protein (for control experiments) is obtained from E. coli strain BL21 (Novagen) transformed with pGEX-2T plasmid.
The test compound (c= 50 nM), e.g. a fluorogenic peptide described in Example 4, is titrated with different amounts of the GST-hdm2 protein (c= 0, 50 nM, 200 nM, and 300 nM). The fluorescence emission spectra (λₑₓ= 370 nm) are recorded in a spectrofluorophotometer at 20°C. The instrument setting during the titration is identical so that the fluorescence intensities in the presence or absence of the GST-hdm2 protein can be compared. A stock solution of the fluorogenic peptide is prepared in a PBS buffer (pH= 7.6) containing 10% glycerol, 1% Triton 100, 50 mM NaCl and BSA (1 mg/ml). The peptide (c= 50 nM) is incubated for 30 min with different amounts of the GST-hdm2 protein. After this time, the fluorescence emission spectrum is recorded. The addition of the GST-hdm2 protein to the solution containing the fluorogenic peptide results in an increase in the emission fluorescence of the fluorogenic peptide at 530 nm. The fluorescence emission spectra of the GST-hdm2 protein (c= 50-300 nM) is identical to the fluorescence emission spectra of the buffer, so the protein does not contribute to the observed increase in emission fluorescence.
This assay is applicable to the detection of specific interactions of peptides or low molecular weight compounds with the MDM2 protein. In addition, it allows accurate kinetic measurements in solution.

### Example 8: identification of MDM2 binding peptides by phage display

In deviation from the previous definition, in the following Examples "hdm2" refers to the human double minute gene2 and the corresponding protein.

### Phage selection

The phage libraries used in this study display random peptide sequences of six, twelve or fifteen amino acid residues at the N-terminus of the minor coat protein III. These libraries are provided by George Smith (University of Missouri, 6 and 15mer) and William Dower (Affymax Research Institute, 12mer). In a biopanning procedure library samples are screened on solid phase GST-hdm2 (hdm2 comprising amino acids 1-188). Polystyrene petri dishes (Falcon 3001) are coated with 5µg/ml GST-hdm2 or DO- 1 (monoclonal anti-p53 antibody) overnight at 4°C in a humidity chamber. Unbound material is washed off with 2.5ml PBS and the dishes are blocked with 2ml TBST-M (150mM NaCl, 50mM Tris-HCl, pH 7.5, 0.1 % (v/v)Tween20, 5% (w/v) low fat milk powder) for 1h at 4 °C. The petri dishes are washed three times with 2.5 ml PBS and 500 µl phage suspension containing 1x10¹¹ (6 and 12mer libraries) or 7.5x10¹² TU (transforming units) is added and allowed to bind for 3h at 4°C. After washing ten times with 2.5ml TBST, bound phages are eluted with 400 µl elution buffer (0.1 N HCl-glycine pH2.2, 1 mg/ml BSA) for 20 min. The eluates are neutralized with 24 µl 2M Tris base and used to infect 2.5ml log-phase E.coli K91 cells (50 min; 37°C). The whole suspension is transfered into 50ml Falcon tubes with 10 ml 2xYT medium containing 20 µg/ml tetracycline (2xYT, Tet) and incubated for 24 hours at 37°C with shaking. The cultures are spun to remove the bacteria and the phage particles are purified from the supernatant by PEG precipitation. The phage pellets are resuspended in 1 ml TBS and aliquots are used for a second round of biopanning which is carried out as for the first with the following modifications:
100 µl amplified phage (2x10¹¹ TU) are reacted with GST-hdm2, MBP-hdm2 or DO-1 which has been absorbed in wells of a 96-well PVC assay plate at a concentration of 5 µg/ml. Amplified and purified phages from this round of biopanning are tested in an ELISA on the proteins used for their selection. For single clone screening E.coli K91 cells are infected with appropriate phage dilutions and spread on LB agar with 20 µg/ml) tetracycline. Single colonies are transfered to wells of a 96-well tissue culture plate containing 200 µm 2xYT medium with 20 µg/ml tetracycline per well. Phage supernatant is collected for ELISA screening after a 24 hour incubation at 37°C with shaking. ELISA positive clones are grown up as 2 ml bacterial cultures in 2xYT medium with 20 µg/ml tetracycline for 24 hours. Phage are PEG precipitated and redissolved in 200 µl TBS buffer. Phage DNA is extracted with phenol/chloroform and ethanol precipitated. The DNA pellet is redissolved in 10 µl water and used as template for sequencing (Sequenase).

### Phage Elisa

### Solid phase hdm2

PVC assay plates are coated with 100 µl antigen (GST-hdm2, MBP-hdm2, GST, MBP, or DO-1) at 5 µg/ml in PBS overnight at 4 °C and blocked with 200 µl PBST-M (PBS, containing 5% (w/v) fat-free dried milk and 0.1%.(v/v) Tween 20) for one hour at room temperature. 100 µl phage suspension (supernatant or PEG concentrated phage) is diluted in PBST-M and added for three hours at 4 °C. Bound phages are reacted with 100 µl HRP-labelled sheep anti-M13 antibody (Pharmacia) for one hour at room temperature followed by substrate development with 100 µl TMB/H202 (0.1 mg/ml TMB, 0.3% H₂O₂ in 0.1 M Na-acetate, pH 6.0) for 15 min. The reaction is stopped by adding 100 µl 1M sulphuric acid to the substrate and the absorbance is measured at 450 nm. All washings between the incubation steps are done with tap water.

### Solution phase hdm2

GST-, MBP-, TRX-hdm2 or baculovirus produced mdm2 (Sf9 cell extract) diluted in PBST-M are reacted in solution with hdm2- or GST-binding phage overnight at 4°C. Simultaneously, ELISA plates are coated with 100 µl Rabbit anti-mouse antibodies (DAKO, Z0259) 1:1000 in 0.1 M NaHCO₃, pH 9.6. The plates are blocked as usual and incubated with monoclonal anti-mdm2 antibodies (hybridoma supernatant 1:5 diluted in PBST-M) for one hour. For the titration ELISA, purified SMP14 (8 µg/ml in PBS) is used to coat the plate directly. In either case, the pre-incubated phage-hdm2 samples are transfered to the coated and blocked plate and incubated for two hours at room temperature. Bound phages are detected as described.

### Results

Phage pools which have been recovered from two rounds of biopanning on solid phase GST- and MBP-hdm2 or DO-1 using samples of 6, 12, or 15mer phage display libraries are screened in ELISAs for antigen binding. Phage from 12 and 15mer libraries selected twice with GST-hdm2 or once with GST-hdm2 followed by MBP-hdm2 clearly bind to GST-hdm2, whereas the 6mer pool is completely negative. On the other hand, monoclonal anti-p53 antibody DO-1 is able to select phages from all three libraries proving the integrity of the 6mer library.
To determine whether phages specific for hdm2 are selected, the phage pools are tested against MBP-hdm2, GST and MBP. Both, 12 and 15mer pools contain hdm2 binding phages (positive for MBP-hdm2, but negative for MBP alone). In addition, 15mer phages twice biopanned with GST-hdm2 (GST/GST-hdm2) are strongly selected for GST-binders. In the 15mer pool which is panned on MBP-hdm2 in the second round (GST/MBP-hdm2) the anti-GST signal is reduced, probably because GST is no longer present as a selecting antigen. GST does not pull out any phage from the 12mer library. Single phage clones are grown from 12 and 15mer pools and tested for GST, GST-hdm2 and MBP-hdm2 binding. Many clones are clearly positive with GST-hdm2 and MBP-hdm2. Phage clones from the 12 and 15mer pools which are shown to be positive with GST- and MBP-hdm2 are selected for further analysis. Phage DNA is extracted from each clone and the nucleotide inserts are sequenced. From 28 clones 6 unique insert sequences are obtained (amino acid sequences given in single letter code):

| | |
|---|---|
| mdm2 binding site on human p53 | P L S Q E T **F** S **D** L **W** K L **L** P E N N V |
| phage clone 12/1 | M P R **F** M D Y **W** E G **L** N |
| phage clone 12/2 | V Q N **F** I **D** Y **W** T Q Q F |
| phage clone 12/5 | T G P A **F** T H Y **W** A T F |
| phage clone 15/1 | I D R A P T **F** R **D** H **W** F A **L** V |
| phage clone 15/5 | P R P A L V **F** A **D** Y **W** E T **L** Y |
| phage clone BB3 | P A **F** S R F **W** S D **L** S A G A H |
| phage consensus | P X **F** X **D** Y **W** X X **L** |

Aligning the corresponding amino acid sequences to each other reveals the phage consensus sequence P X F X D Y W X X L. It shows similarity to the known mdm2 binding motif on p53, T F S D L W (amino acid residues 18-23 of human p53; Picksley et al., Oncogene 9, 2523-2529 (1994) which reference is incorporated herein by reference in its entirety. All phage sequences contain the phenylalanine (F) and Tryptophan (W), and 4 out of 6 the aspartic acid (D) and leucine (L) found in the same position in the mdm2 motif. A strong selection for tyrosine (Y) and proline (P) in the phage sequences is not met by corresponding residues in the p53 sequence, although 2 successive prolines are found further upstream of the mdm2 binding site.
Further experiments are designed to evaluate the specificity of the hdm2-phage interaction and to prove that p53 and hdm2 phage bind to the same region on hdm2. For these experiment clones BB2/BB11 (GST binding control phage) and BB3/BB10 (hdm2 phage) are chosen. Phages are preincubated in solution with GST-hdm2, MBP-hdm2 or TRX-hdm2 and the phage-hdm2 complexes are transfered to wells which contain different monoclonal anti-mdm2 antibodies bound to the solid phase via anti-mouse antibodies. Bound phages are detected. As expected, GST-phages are able to bind only to GST-hdm2. All three anti-mdm2 antibodies used (SMP14, 4B2, 3G5) are able to bind to hdm2 complexed with GST-phage. Antibody SMP14 is commercially available and described in Picksley et al., Oncogene 9, 2523-2529 (1994) incorporated by reference above. Antibodies 3G5 and 4B2 are described in Chen, J. et al., Mol.Cell Bio., vol. 13, 4107-4114 (1993) also incorporated herein by reference in its entirety. On the other hand, the hdm2-phage recognizes all three hdm2 fusion proteins including TRX-hdm2 never used for the phage selection (biopanning). The hdm2-phage complexes are efficiently pulled down by SMP14 and 4B2, but 3G5 is hardly able to bind to these complexes. It has been shown that mdm2-p53 complexes cannot be bound by 3G5, probably because the epitope of this antibody lies within the p53 binding domain of mdm2. Another experiment shows that hdm2-phages but not GST-phages are able to inhibit the interaction between hdm2 and TIP. TIP is thioredoxin with the mdm2 binding sequence of p53 inserted into its active site. In order to estimate the relative affinities of the different phage clones towards hdm2, a dilution series of GST-hdm2 is offered in solution to a fixed amount of phages. Phage-hdm2 complexes are pulled down by solid phase SMP14 and bound phages are detected. All phage clones tested show a very similar strong binding to GST-hdm2 with a half-maximal binding concentration of 0.5 to 10 nM GST-hdm2, dependent on the hdm2 preparation used. Experiments with baculovirus produced mdm2 (Sf9 cell extract) prove that the phage clones selected with hdm2 are able to bind to its mouse homologue as well.
The phage sequences and a consensus sequence are produced as free peptides and tested for their relative capacity to block the interaction of MDM2 with p53 in three different ELISA formats. The new consensus sequence and some of the phage derived peptides show a remarkable increase in specific activity over the wild type p53 peptide sequence.

### Materials and Methods for protein expression

### 1. Thioredoxin(Thio)-fusions

The clones are produced using the Invitrogen-Expression system. Using Bluescript containing the hdm2 gene as a template, PCR is carried out with the following primers (5'-3'):
START2 primer: gcg gat ccg atg gtg agg age agg caa atg
STOP1 (to N221): gcc tgc age cta att cga tgg cgt ccc tgt aga
STOP2 (full length): gc ctg cag cta ggg gaa ata agt tag cac aat
STOP3 (to D294): gc ctg cag cta atc ttc ttc aaa tga atc tgt
START3 primer (from D294): ggg gat cct gaa att tcc tta gct gac.

The resulting PCR products are cloned into pCR II (TA cloning kit, Invitrogen). The resulting plasmids are cleaved with BamH1 and PstI. The products are ligated into the BamH1/PstI cleaved pTrxFus. The plasmid is introduced into E.coli G1724. The following clones are obtained:
1. clone 1/8 Thio-MVRSRQ-MI...N221
2. clone 3/8 Thio-MVRSRQ-MI...D294
3. clone 2/7 Thio-MVRSRQ-MI...C478

### 2. Maltose binding protein (MBP)-fusion

The PCR product is obtained using the STOP1 primer described above with START1: gc gga tcc atg gtg agg age agg caa atg.
It is again cloned into pCR II. The plasmid is cut with BamH1 and Pstl. The products are ligated into BamH1/Pstl cleaved pMALc2 (New England Biolabs). The plasmids are then introduced into E.coli INV(F' cells (One ShotTMcompetent cell kit, Invitrogen).
Clone 4 (MBP-MVRSRQ-M1...N221) is obtained.

### 3. GST-fusion protein

A plasmid containing wild type hdm2 is used as a template in PCR. The primers are designed such that a BamH1 site is introduced into the 5' end and a EcoR1 site into the 3' end of the gene. The PCR products are digested and ligated into pGEX-2T (Pharmacia). The plasmid is then introduced into E.coli BL21 cells.

### Protein expression:

### 1. Thioredoxin-fusionproteins

Cells are grown in RM medium (1 xM9 salts, 2% Casamino acids, 1 % glycerol, 1mM MgCl₂, 100 g/ml ampicillin) overnight at 30°C. They are then inoculated into fresh Induction medium (1x M9 salts, 0.2 % Casamino acids, 0.5 % glucose, 1 mM MgCl₂, 100 g/ml ampicillin to a dilution of 1/20. Cells are then grown to an OD of 0.25 to 0.5 at 30°C. The culture is transfered to 37°C and induced with L-Tryptophan at a final concentration of 100 g/ml. After 3 h cells are harvested by centrifugation. The pellets are resuspended in ice cold 20 mM Tris/HCl, pH 8, 2.5 mM EDTA with protease inhibitors (1 mM PMSF, 1 mM benzamidine, leupeptin, approtinin and pepstatin at 10 g/ml each. The cells are lysed by sonication, shock freezing, quick thawing. The cycle is repeated two more times. The lysate is then centrifuged at 4000 rpm for 15 min at 4°C. The supernatant is used.

### 2. Maltose binding protein-fusions

Cells are grown in rich Medium with glucose and ampicillin (10 g tryptone, 5 g yeast extract, 5 g NaCl, 2 g glucose and 100 g/ml ampicillin to an OD of 0.5. They are then induced with IPTG at a final concentration of 0.3 mM. Incubation is continued at 37°C for another 2 h. Cells are harvested by centrifugation and resuspended in column buffer (1/20th of original volume, 20 mM Tris-HCl, pH 7.4; 200 mM NaCl; 1 mM EDTA; 1 mM DTT plus protease inhibitors as mentioned above. Cells are frozen over night at -20°C. They are thawed in cold water, sonicated on ice in short pulses of 6 x 10 seconds and spun at 9000 rpm for 30 min at 4°C. The supernatant is diluted 1/5 with column buffer and loaded on an amylose resin (New England Biolabs, 15 ml, prepared in column buffer). Elution is carried out with column buffer + 10 mM Maltose. Active fractions are pooled, concentrated and desalted on a 10 DG column (BioRad). They are stored in 50 mM Tris /Hcl pH 7.4, 50 mM NaCl, 20 % glycerol, 1 mM DTT.

### 3. GST-hdm2 (1-188)

Bacteria cultures are grown to OD 0.8. They are cooled to RT and induced with 1 mM IPTG, then grown for 4h at 27°C. Cells are harvested and pellets flash frozen in liquid nitrogen. Pellets are resuspended in ice cold buffer A (0.5 M NaCl, 2.7 mM KCl, 10 mM Na₂HPO₄, 1.8 mM KH₂PO₄, 1 mM PMSF, 1 mM EDTA, 10 mM 2-mercaptoethanol, pH 7.3). They are lysed by a French press or alternatively by sonication. After centrifugation the soluble fraction is loaded onto a glutathione sepharose 4B column (Pharmacia) equilibrated with buffer A. The protein is then eluted with buffer B (50 mM Tris/HCl, 10 mM reduced glutathione, 0.5 M NaCl, 1 mM EDTA. 1 mM PMSF or benzamidine, 10 mM 2-mercaptoethanol or 1 mM DTT, pH 8.0.) Active fractions are desalted on Sephadex G25 or 10 DG (BioRad) preequilibrated with buffer C (50 mM Tris/HCl, 50 mM NaCl, 20 % glycerol, 10 mM 2-mercaptoethanol or 1 mM DTT, 0.1 % Triton x-100, pH 7.6). The protein is used for Elisas or further purified on a Mono Q column (Pharmacia) preequilibrated with buffer C. The protein is eluted with a linear gradient of buffer C containing 1 M NaCl. The fractions containing fusionprotein are pooled, concentrated (Centricon 30), flash frozen in liquid nitrogen and stored at - 70°C.

### ELISAs

Three different Elisas are employed to analyze the interaction between hdm2 and p53.
They are named according to the reagent which is used to coat the Elisa plates. All Elisas are carried out at 4°C.

### 1. Elisa P2

P2 is a biotinylated peptide consisting of 18 amino acids of the Terminal part of p53, namely: Biotin-S-G-S-G-E-P-P-L-S-Q-E-T-F-S-D-L-W-K-L-L-P-E
Plates are incubated overnight with 10 µg/ml streptavidin at 37°C. They are blocked with 2% BSA in PBS for 1 h. Peptide is applied at 5 µg/ml in blocking solution for 1 h. A second blocking step is carried out with 5 % milk, 0.1 % Tween20 in PBS (blocking solution2) for a minimum of 10 min. Hdm2fusionproteins are diluted in blocking solution2 and applied for 1h. Bound hdm2 is detected with SMP 14 hybridoma cell supernatant diluted 1/2 in block solution2. HRP-anti-mouse IgG (DAKO) is used as second antibody. Washing between incubations is carried out 6 times with tap water.

### 2. Elisa TIP

TIP is thioredoxin which has additional amino acids inserted into its active site. These are derived from the N-terminus of p53 and are the following:
P-P-L-S-Q-E-T-F-S-D-L-W-K-L-L-P-E-N.
The following are used
P1: 5' gt ccg cct ctg agt cag gaa aca ttt tca gac cta tgg aaa cta ctt cct gaa aac g 3'
P2: 5' g acc gtt ttc agg aag tag ttt cca tag gtc tga aaa atg ttt cct gac tca gag gcg 3' 10 ng of each P1 and P2 are phosphorylated using PNK and annealed for 1 h at 37°C. The vector pTRX (InVitrogen) is cleaved with RsrII and dephosphorylated. After ligation the plasmids are introduced into E.coli 1724 cells.
Plasmid containing bacteria are grown in RM medium at 30°C and induced with L-Trp as described earlier. A soluble lysate is made by freeze -thaw-sonication cycles. This lysate is then heat shocked at 80°C for 10 min. The soluble fraction is used to coat Elisa-plates at a concentration of 40 µg/ml in PBS o/n.Plates are blocked in blocking solution2 for 1 h. Incubation with hdm2 fusionproteins and detection is carried out as described for Elisa P2.

### 3. Elisa hdm2

Plates are coated with 2.6 µg/ml GST-hdm2(1-188) in PBS at 4°C o/n. They are blocked in blocking solution 2 for 1 h. Full length p53, lysozyme lysate from E. coli, purified on heparin-sepharose is applied in blocking solution2 with 10 % glycerol and 10 mM DTT for 1h.
Binding is established with mAb 421 and HRP coupled anti mouse IgG.
HRP activity is measured using TMB. For inhibition Elisas inhibitors are preincubated with either hdm2-fusionproteins or p53 for 15 min before the mixture is transfered to the plate. Peptide inhibitors are dissolved in DMSO.

### Example 9: Purification of p53 D30

The human wild type p53 gene is used as a template for PCR to obtain the gene fragment encoding for residues 1 to 362 of the 393 amino acids of natural (human) p53 (p53D30). The oligonucleotides used for PCR are designed such that a Ndel restriction site is introduced at the 5' end and a BamHl site at the 3' end. The PCR fragments digested by Ndel and BamHl are ligated with a NdeI/ BamHI cleaved pET-3a plasmid. The complete gene is sequenced and the expression plasmid is introduced into *E. coli* strain BL21 (DE3)pLysS (Novagen).
For protein expression bacteria cultures are inoculated by a 100-fold diluted overnight culture and grown in Luria Broth medium in the presence of 100 µg ampicillin/ ml at 37 °C to OD₆₀₀ = 0.8. The cultures are then cooled on ice to room temperature, induced with 1 mM isopropyl-D-thiogalactopyranoside and grown for four additional hours at 27°C. The cells are then harvested by centrifugation and the pellets flash frozen in liquid nitrogen and stored at -70 °C.
The cell pellets containing the p53D30 protein are resuspended in ice cold buffer D (50 mM 4-(2-hydroxyethyl)-piperazine-ethane-sulfonic acid (Hepes.NaOH), 10 % (v/v) glycerol, 0.1 mM EDTA, 0.1 % (v/v) Triton X-100, 5 mM 1,4-dithio-DL-threitol (DTT), 1 mM PMSF - pH = 7.6) and lysed with a French press at 1000 psi. After centrifugation, the soluble fraction is loaded onto a HiTrap Heparin column (Pharmacia Biotech) preequilibrated at 4 °C with buffer D. The column is first washed with buffer D containing 22 % buffer E (50 mM Hepes.NaOH, 1 M KCl, 10 % (v/v) glycerol - pH = 7.6) and p53D30 is eluted with a linear gradient to 100 % buffer E. The fractions containing p53D30 are pooled and loaded onto a HiTrap metal chelation column (Pharmacia Biotech) charged with nickel and preequilibrated at 4 °C with buffer F (50mM Hepes.NaOH, 0.5 M KCl, 10 % (v/v) glycerol - pH = 7.6). After washing the column with buffer F containing 20 % buffer G (50 mM Hepes.NaOH, 0.5 M KCl, 10 % (v/v) glycerol, 0.1 M immidazole - pH = 7.6), p53D30 is eluted with 45 % buffer G. 50 mM 2-mercaptoethanol and 1 mM ZnCl₂ are added to the solution and the protein is dialysed at 4 °C against 50 mM Hepes.NaOH, 0.5 M KCl, 20 % (v/v) glycerol, 50 mM 2-mercaptoethanol, 1 mM ZnCl₂ - pH = 7.6. p53D30 is concentrated to 1 mg/ml (Amicon 30 kDa cut off membrane), flash frozen in liquid nitrogen and stored at -70 °C. Protein analysis

The purity of the protein preparation is evaluated by gel scanning (Schimadzu CS-930) on a SQS-PAGE (Laemmli, U.K. (1970) Nature, 227, 680-385) stained with Coomassie blue. Protein concentration is determined according to Bradford, M.B. (1976) Anal. Biochem., 72, 248-254).

### Example 10:

To improve the intracellular stability and facilitate cellular uptake of the peptides described in examples 1 to 9, peptide binding elements may be constructed in which the peptides of the present invention are presented on the active site of Escherichia coli thoredoxin. The pTrx vector (Invitrogen) is cleaved with restriction enzyme RsrII. Ogligomers, corresponding to the peptide identified on clone 12/1, described in example 8 above, and wild type p53 sequences are phosphorylated, annealed and then ligated into the cleaved pTrx vector.

The following oligomers may be use to produce a binding element (TIP wt) comprising a p53 wild type peptide insert:

5'-3'
GTCCGCCTCTGAGTCAGGAAACATTTTCAGACCTATGGAAACTACTTCCTGA
AAACG, and 5'- 3'

GACCGTTTTCAGGAAGTAGTTTCCATAGGTCTGAAAATGTTTCCTGACTCAG
AGGCG

The following oligomers may be used to produce a binding element (TIP 12/1) comprising the peptide insert of clone 12/1 described in example 8.

5'-3':
GTCCGCCTCTGAGTATGCCTCGTTTTATGGATTATTGGGAGGGTCTTAATGA
AAACG amd 5'-3-

GACCGTTTTCATTAAGACCCTCCCAATAATCCATAAAACGAGGCATACTCTC
AGAGGCG.

E.Coli 1724 cells are transformed with the resulting plasmids as well as pTrx which may act as a negative control binding element (Trx) comprising thioredoxin without a peptide insert. The cultures may be grown in RM medium (1 xM9 salts, 2% Casamino acids, 1 % glycerol, 1 mM MgCl₂, 100 g/ml ampicillin) overnight at 30°C. The cultures are inoculated into fresh induction medium (1x M9 salts, 0.2 % Casamino acids, 0.5 % glucose, 1 mM MgCl₂, 100 g/ml ampicillin to a dilution of 1/20) and grown to an Optical Density (OD) of 0.25 to 0.5 at 30°C. The culture is transferred to 37°C and induced with L-Tryptophan at a final concentration of 100 g/ml. After 3 hours to 4 hours cells are harvested by centrifugation. The pellets are resuspended in ice cold 20 mM Tris/HCl, pH 8,2.5 mM EDTA with protease inhibitors 1 mM PMSF, 1 mM benzamidine, leupeptin, approtinin and pepstatin at 10 g/ml each. The cells are lysed by shock freezing, thawing and sonicating. The cycle is repeated two more times. The soluble lysate is then centrifuged at 10000g for 20 min at 4°C. Heat shock lysates are obtained by resuspending petllets to an OD of 100 and then treating at 80°C for 10 minutes followed by centrifugation at 10,000g for 20 min.

Purification of soluble extracts is carried out by loading clear soluble lysates onto an Ion exchange Q50 column (BioRad) and eluting with a linear gradient of 0.05M-1 MKCL in 50mMTris/HCL pH7.8, 0.1%Triton X-100, 10% glycerol and 50mMKCL.

Active fractions may be identified on dot blots with an anti-thioredoxin antibody available from Invitrogen. The active fractions may then be concentrated using Centriprep 3 filters (Amicon) and loaded unto a G100 column (Pharmacia) which has been preequilibrated with 30 mM HEPES, pH 8.0, 500 mM KCL, 0.1% Triton X100, and 10% glycerol. Following elution, active fractions may be pooled, concentrated and dialyzed against PBS.

For expression in mammalian cells, the complete thioredoxin coding region with peptide insertions (TIP wt, and TIP 12/1) or without peptide inserts (Trx) may be PCR amplified using standard PCR reagents and conditions known in the art and the following primers: 5'-3': CGGGATCCACCATGGGCGATAAAATTATTCACCTG and 5'-3': CTCGACGCTAACCTGGCCTAGGGAATTCC.

The resulting PCR products may be cleaved with BamHl and Eco RI and ligated into BamHl and EcoRl cleaved pcDNA3. pcDNA3 (Promega) is a vector having a CMV promoter for driving expression of TIP wt, TIP 12/1 and Trx in mammalian cells. The plasmids may be amplified in E. coli as known in the art. Plasmid DNA encoding for TIP 12/1, TIP wt and Trx may be purified using a Quiagent purification system or phenol/chloroform precipitation.

Antibodies or DNA encoding the described binding elements may be microinjected into Vrn.6 cells, a transformed rat thyroid epithelial cell line and T22 cells, a mouse prostrate derived cell line both cell lines being stably transfected with pRGC ΔFos-Lacz, a p53 responsive β-galactosidase reporter. Production of the Vrn.6 cell line and the pRGCΔFos-Lacz reporter are known in the art. Blaydes, J.P. et al., (1997), Oncogene, vol 14, in press; and Hupp, T.R et al. (1995) Cell, vol. 83, 237-245 hereby incorporated by reference in its entirety. Vrn.6 tolerate hight levels of wild type p53 and overexpress MDM2 at a protein level. T22 cells typically contain low levels of p53 and mdm2.

For microinjection, cells may be seeded into tissue culture dishes and grown to 60-70% confluence. Microinjection may be performed using an Eppendorfer micorinjection system (Microinjector 5242, Micromanipulator 5170) mounted to an Axiovert light microscope (Zeiss) having a heated stage.

Purified mouse monoclonal antibodies 3G5 and 4B2 may be injected intranuclearly and cytoplasmicly in PBS at a concentration of 1.3 mg/ml. Plasmid DNA encoding for TIP 12/1, TIP wt and Trx may be injected intronuclearly in water at a concentration of 0.25 mg7ml. Following microinjection, fresh medium may be added to the cell cultures and the cultures further incubated for 24 hours.

To detect β-galactosidase activity, cells may be washed with PBS and fixed with 2% formaldehyde,/0.2% glutaraldehyde in PBS for 5 minutes on ice. The cells may be washed again and overlaid with X-gal (0.25 mg/ml) in a reaction mix (5mM potassium ferricyanide, 2mM magnesium chloride in PBS). Cells may then be incubated at 37 °C for 16 hours after which they may be observed for blue stained cells indicating a positive response.

### Results:

In Vrn.6 cells, having overexpressed MDM2, a positive response was observed when 3G5 antibody or TIP 12/1 were injected intranuclearly. There was not a positive response weht Trx was injected intranuclearly. 3G5 binds mdm2 withing the p53 binding pocket thereby blocking p53-MDM2 association (Böttinger et al., 1997)

In T22 cells, a low level p53 and mdm2 containing cell line, a strong positive response was observed when 3G5 and TIP 12/1 were injected. A positive but lower level response was observed whent TlPwt was injected. No response was observed when 4B2 antibody or Trx were injected. 4B2 is an anti MDM2 antibody that targets an epitope outside the p53 binding pocket on MDM2.

DNA encoding the described binding elements TIP 12/1, TIP wt and Trx and the pRGCΔFosLacZ reporter may be transiently transfected into the following three different cell types, OSA cells, a human osteosarcome cell line, U2-Os cells, another osteosarcoma cell line, and MCF-7 cells, a breast conacer cell line containing wild type p53. The OSA cell line contains a highly elevated mdm2 level due to gene amplification (Florence et al. 1994). The U2-OS cell line has no gene amplification for mdm2 but has elevated levels of mdm2-mRNA (Florence et al. 1994). The MCF-7 cell line contains heterogenously expressed low levels of wild type p53 and no reported mdm2 elevation.

For transient transfection and reporter induction, cells are seeded into 6 well plates at 1.5 x 10⁶ cell per well. They are grown to a density of 80% confluence and transfected using different Lipophilic reagents such Lipofectin and Lipofectamin from Promega or Dosper and Dotap from Boehringer. 2.5 µg of TIP encoding plasmid DNA, 1 µg RGCΔFosLacZ DNA and 5-10µg of lipophili reagent according to manufacturer instructions are mixed in serum free medium and applied to the cells. Two to four hours after transfection, complete medium is added. Forty-eight hours after transfection β-galactosidase activity is measured using DPRG (Boehringer) as a substrate. Cells are scraped into PBS and centrifuged. Pellets from each well are dissolved in 50 µl of Reporter Lysis buffer (Promega) and incubated on ice for 15 minutes. Soluble Lysates are incubated with CPRG in 100 mM phosphate buffer, pH 7.0. Optical Density at 595 nm is measured 1 to 24 hours later.

Results: Surprisingly, most induction of the p53 reporter is achieved by TIP12/1 in MCF-7 cells and in U2-OS cells. Lower induction is observed in TIP 12/1 transfected OSA cells. Transfection of the control plasmid alone induces a lowe level respons of p53 dependent transcriptional activation in MCF-7 and U2-OS cells but is almost completely absent in OSA cells.

T22 cells, U2-Os cells, OSA cells and SAOS 2 cells may be grown in in Dulbeccor's modified Eagle medium (DMEM) supplemented with 10% Fetal Calf Serum. Additionally, for T22 cells 1 mg/ml of the antibiotic G418 may be added. Vrn. 6 cells are grown as is known in the art, previously described by Blaydes et al., 1997.

## Claims

1. A compound capable of binding to MDM2 and inhibiting or blocking the binding of MDM2 to p53 protein *in vitro* or *in vivo,* the compound being superior to the peptide having the amino acid sequence QETFSDLWKLLP corresponding to the correct p53 wild-type sequence in its ability to selectively inhibit the binding of p53 and MDM2, wherein the compound is either
(i) a peptide comprising an amino acid motif of the formula
R₁-X₁-F-X₂-R₂ -R₃-W-X₃-X₄-R₄ (I),
wherein
R₁ is proline (P), leucine (L), glutamic acid (E), cysteine (C) or glutamine (Q),
X stands for any natural amino acid,
R₂ is arginine (R), histidine (H), glutamic acid, cysteine, serine, or preferably aspartic acid (D),
R₃ is histidine (H), phenylalanine (F) or tyrosine (Y),
R₄ is phenylalanine (F), glutamine (Q) or leucine (L), and
F is phenylalanine and W is tryptophan; or
(ii) a fragment of peptide (I) comprising at least the eight consecutive amino acids
F-X₂-R₂-R₃-W-X₃-X₄-R₄ (Ib)
from the sequence of formula (I)
wherein the peptide (I) or (Ib) is modified by substitution such that:
R₂, X₃ and/or X₄ are, independently from one another, replaced by α,α-disubstituted amino acid residue, α-aminoisobutyric acid, 1-amino-cyclopropane-1-carboxylic acid, 1-amino-cyclopentane-1-carboxylic acid, 1-amino-cyclohexane-1-carboxylic acid, 4-amino piperidine-4-carboxylic acid, or 1-amino-cycloheptane-1-carboxylic acid,
and wherein the modification is such that an α-helix conformation in the peptide is induced, increased or maintained.

2. A compound according to claim 1, wherein the MDM2 is human DM2.

3. A compound according to any preceding claim, wherein the p53 is human p53.

4. A compound according to any preceding claim, wherein R₂ is α-aminoisobutyric acid (Aib), X₃ is α-aminoisobutyric acid and/or X₄ is 1-amino-cyclopropane-1-carboxylic acid (Ac₃c).

5. A compound according to any one of the preceding claims consisting of no more than fifteen amino acids (15mers).

6. A compound according to any one of the preceding claims wherein the peptide comprising an amino acid motif of formula (I) is selected from the group consisting of peptides with the sequences M-P-R-F-M-D-Y-W-E-G-L-N, Q-P-T-F-S-D-Y-W-K-L-L-P, and P-X-F-X-D-Y-W-X-X-L.

7. A compound according to any one of claims 1 to 5 wherein for
F-X₂-R₂-R₃-W-X₃-X₄-R₄ (II)
X₂ is methionine, isoleucine, threonine, arginine, alanine or serine, preferably methionine;
X₃ is glutamic acid, threonine, alanine, phenylalanine or serine, preferably glutamic acid; and
X₄ is glycine, glutamine, threonine, alanine or aspartic acid, preferably glycine.

8. A compound according to any one of claims 1 to 6 wherein the fragment has the formula
X₁-F-X₂-R₂-R₃-W-X₃-X₄-R₄ (Ic),
wherein R₁, R₂, R₃ and R₄, independently from one another, each have the meanings and preferences given for formula (I),
X₁ is arginine, asparagine, alanine, threonine or valine; particularly arginine
X₂ is methionine, isoleucine, threonine, arginine, alanine or serine; preferably methionine;
X₃ is glutamic acid, threonine, alanine, phenylalanine or serine; preferably glutamic acid; and
X₄ is glycine, glutamine, threonine, alanine or aspartic acid, preferably glycine.

9. A compound of any preceding claim carrying one or more protecting groups.

10. A compound of any preceding claim in the form of a salt.

11. A compound of any preceding claim wherein the peptide is in cyclic form.

12. A compound according to claim 11, which contains a disulphide bridge, a thioether bridge or a lactam.

13. A conjugate comprising the compound of any preceding claim covalently attached to another peptide or protein.

14. A conjugate comprising a compound of any one of claims 1 to 12, labeled directly or by way of a spacer or linker group with an enzyme, a fluorescent marker, a chemiluminescent marker, a metal chelate, paramagnetic particles or biotin.

15. Use of a compound or conjugate according to any preceding claim for the identification of a molecule binding to MDM2.

16. Use of a compound or conjugate according to any of claims 1 to 14 for the purification of a binding partner, particularly MDM2.

17. Use of a compound or conjugate according to any of claims 1 to 14 in a method aiming at identifying or designing compounds which interfere with the binding of MDM2 to p53.

18. Use of a compound or conjugate according to any of claims 1 to 14 for *in vitro* diagnosis of a disease.

19. A pharmaceutical composition that is suitable for administration to a warm-blooded animal, including humans, or to cells or cell lines derivable from a warm-blooded animal, including a human, for the treatment or prevention of a disease that responds to inhibition of the interaction of p53 with MDM2, said composition comprising an amount of a compound or conjugate according to any of claims 1 to 14, which is effective for said inhibition, together with at least one pharmaceutically acceptable carrier.

20. A process for the preparation of a compound or conjugate according to any of claims 1 to 14 comprising reacting a fragment of such peptide, which has a free carboxy group, or a reactive derivative thereof, with a complementary fragment that has an amino group with at least one free hydrogen atom, or with a reactive derivative thereof, resulting in the formation of a peptide bond, and, if desired, removing a present protecting group, or derivatising said peptide or derivative.

21. A compound or conjugate according to any of claims 1 to 14, for use in a method of treatment.

22. A compound or conjugate according to claim 21, wherein the treatment is of hyperproliferative disease.

23. A compound or conjugate according to claim 22, wherein the treatment is of tumour.

24. The use of a compound or conjugate according to any of claims 1 to 14 for the preparation of a pharmaceutical composition for the treatment or prevention of hyperproliferative disease.

25. Use according to claim 24, wherein the treatment is of tumour.

## Patentansprüche

1. Verbindung, die in der Lage ist, an MDM2 zu binden und die Bindung von MDM2 an p53-Protein zu inhibieren oder zu blockieren, in vitro oder in vivo, wobei die Verbindung gegenüber dem Peptid mit der Aminosäuresequenz QETFSDLWKLLP, die der korrekten p53-Wildtyp-Sequenz entspricht, in ihrer Fähigkeit, die Bindung von p53 und MDM2 selektiv zu inhibieren, überlegen ist, worin die Verbindung entweder
(i) ein Peptid ist, das ein Aminosäuremotiv der Formel
R₁-X₁-F-X₂-R₂-R₃-W-X₃-X₄-R₄ (I)
umfasst,
worin
R₁ Prolin (P), Leucin (L), Glutaminsäure (E), Cystein (C) oder Glutamin (Q) ist, X für jede beliebige natürliche Aminosäure steht,
R₂ Arginin (R), Histidin (H), Glutaminsäure, Cystein, Serin oder vorzugsweise Asparaginsäure (D) ist,
R₃ Histidin (H), Phenylalanin (F) oder Tyrosin (Y) ist,
R₄ Phenylalanin (F), Glutamin (Q) oder Leucin (L) ist und
F Phenylalanin ist und W Tryptophan ist; oder
(ii) ein Fragment von Peptid (I) ist, das zumindest die acht aufeinander folgenden Aminosäuren
F-X₂-R₂-R₃-W-X₃-X₄-R₄ (Ib)
aus der Sequenz aus Formel (I) umfasst,
worin das Peptid (I) oder (Ib) durch Substitution so modifiziert ist, dass:
R₂, X₃ und/oder X₄ unabhängig voneinander durch einen α,α-disubstituierten Aminosäurerest, α-Aminoisobuttersäure, 1-Aminocyclopropan-1-carbonsäure, 1-Aminocyclopentan-1-carbonsäure, 1-Aminocyclohexan-1-carbonsäure, 4-Aminopiperidin-4-carbonsäure oder 1-Aminocycloheptan-1-carbonsäure ersetzt sind,
und worin die Modifikation eine solche ist, dass eine α-Helixkonformation im Peptid induziert, erhöht oder beibehalten wird.

2. Verbindung nach Anspruch 1, worin das MDM2 menschliches DM2 ist.

3. Verbindung nach einem der vorangegangenen Ansprüche, worin das p53 menschliches p53 ist.

4. Verbindung nach einem der vorangegangenen Ansprüche, worin R₂ α-Aminoisobuttersäure (Aib) ist, X₃ α-Aminoisobuttersäure ist und/oder X₄ 1-Aminocyclopropan-1-carbonsäure (Ac₃c) ist.

5. Verbindung nach einem der vorangegangenen Ansprüche, bestehend aus nicht mehr als fünfzehn Aminosäuren (15mere).

6. Verbindung nach einem der vorangegangenen Ansprüche, worin das Peptid, das ein Aminosäure-Motiv der Formel (I) umfasst, aus der aus den Peptiden mit den Sequenzen M-P-R-F-M-D-Y-W-E-G-L-N, Q-P-T-F-S-D-Y-W-K-L-L-P und P-X-F-X-D-Y-W-X-X-L bestehenden Gruppe ausgewählt ist.

7. Verbindung nach einem der Ansprüche 1 bis 5, worin für
F-X₂-R₂-R₃-W-X₃-X₄-R₄ (II)
X₂ Methionin, Isoleucin, Threonin, Arginin, Alanin oder Serin, vorzugsweise Methionin, ist;
X₃ Glutaminsäure, Threonin, Alanin, Phenylalanin oder Serin, vorzugsweise Glutaminsäure, ist; und
X₄ Glycin, Glutamin, Threonin, Alanin oder Asparaginsäure, vorzugsweise Glycin, ist.

8. Verbindung nach einem der Ansprüche 1 bis 6, worin das Fragment die Formel
X₁-F-X₂-R₂-R₃-W-X₃-X₄-R₄ (Ic)
aufweist,
worin R₁, R₂, R₃ und R₄ unabhängig voneinander jeweils die für Formel (I) angegebenen Bedeutungen und Präferenzen aufweisen,
X₁ Arginin, Asparagin, Alanin, Threonin oder Valin, insbesondere Arginin, ist;
X₂ Methionin, Isoleucin, Threonin, Arginin, Alanin oder Serin, vorzugsweise Methionin, ist;
X₃ Glutaminsäure, Threonin, Alanin, Phenylalanin oder Serin, vorzugsweise Glutaminsäure, ist; und
X₄ Glycin, Glutamin, Threonin, Alanin oder Asparaginsäure, vorzugsweise Glycin, ist.

9. Verbindung nach einem der vorangegangenen Ansprüche, die eine oder mehrere Schutzgruppen aufweist.

10. Verbindung nach einem der vorangegangenen Ansprüche in der Form eines Salzes.

11. Verbindung nach einem der vorangegangenen Ansprüche, worin das Peptid eine zyklische Form aufweist.

12. Verbindung nach Anspruch 11, die eine Disulfidbrücke, eine Thioetherbrücke oder ein Lactam enthält.

13. Konjugat, umfassend die Verbindung nach einem der vorangegangenen Ansprüche, die kovalent an ein anderes Peptid oder Protein gebunden ist.

14. Konjugat, umfassend eine Verbindung nach einem der Ansprüche 1 bis 12, direkt oder durch einen Spacer oder eine Linkergruppe mit einem Enzym, einem fluoreszierenden Marker, einem chemilumineszierenden Marker, einem Metallchelat, paramagnetischen Partikeln oder Biotin markiert.

15. Verwendung einer Verbindung oder eines Konjugats nach einem der vorangegangenen Ansprüche zur Identifikation eines an MDM2 bindenden Moleküls.

16. Verwendung einer Verbindung oder eines Konjugats nach einem der Ansprüche 1 bis 14 zur Reinigung eines Bindungspartners, insbesondere von MDM2.

17. Verwendung einer Verbindung oder eines Konjugats nach einem der Ansprüche 1 bis 14 in einem Verfahren, das der Identifikation oder dem Design von Verbindungen dient, welche die Bindung von MDM2 an p53 stören.

18. Verwendung einer Verbindung oder eines Konjugats nach einem der Ansprüche 1 bis 14 zur In-vitro-Diagnose einer Krankheit.

19. Pharmazeutische Zusammensetzung, die zur Verabreichung an ein warmblütiges Tier, unter anderem an einen Menschen, oder an Zellen oder Zelllinien, die von einem warmblütigen Tier, unter anderem einem Menschen, erhältlich sind, zur Behandlung oder Prävention einer Erkrankung geeignet ist, die auf die Inhibierung der Wechselwirkung von p53 mit MDM2 reagiert, wobei die Zusammensetzung eine Menge einer Verbindung oder eines Konjugats nach einem der Ansprüche 1 bis 14, die für die Inhibierung wirksam ist, zusammen mit zumindest einem pharmazeutisch annehmbaren Träger umfasst.

20. Verfahren zur Herstellung einer Verbindung oder eines Konjugats nach einem der Ansprüche 1 bis 14, umfassend das Umsetzen eines Fragments dieses Peptids, das eine freie Carboxygruppe aufweist, oder eines reaktiven Derivats davon mit einem komplementären Fragment, das eine Aminogruppe mit zumindest einem freien Wasserstoffatom aufweist, oder mit einem reaktiven Derivat davon, was zur Bildung einer Peptidbindung führt, sowie, falls gewünscht, das Entfernen einer vorhandenen Schutzgruppe oder das Derivatisieren des Peptids oder Derivats.

21. Verbindung oder Konjugat nach einem der Ansprüche 1 bis 14 zur Verwendung in einem Behandlungsverfahren.

22. Verbindung oder Konjugat nach Anspruch 21, worin es sich bei der Behandlung um die Behandlung einer hyperproliferativen Erkrankung handelt.

23. Verbindung oder Konjugat nach Anspruch 22, worin es sich bei der Behandlung um die Behandlung eines Tumors handelt.

24. Verwendung einer Verbindung oder eines Konjugats nach einem der Ansprüche 1 bis 14 zur Herstellung einer pharmazeutischen Zusammensetzung zur Behandlung oder Prävention einer hyperproliferativen Erkrankung.

25. Verwendung nach Anspruch 24, worin es sich bei der Behandlung um die Behandlung eines Tumors handelt.

## Revendications

1. Composé capable de liaison à MDM2 et d'inhibition
ou de blocage de la liaison de MDM2 à la protéine p53 *in vitro* ou *in vivo*, le composé étant supérieur au peptide ayant la séquence d'acides aminés QETFSDLWKLLP correspondant à la séquence correcte du type sauvage de p53 dans son aptitude à inhiber sélectivement la liaison de p53 et de MDM2, où le composé est soit
(i) un peptide comprenant un motif d'acides aminés de la formule :
R₁-X₁-X₂-R₂-R₃-W-X₃-X₄-R₄ (I),
où
R₁ est proline (P), leucine (L), acide glutamique (E), cystéine (C) ou glutamine (Q),
X indique tout acide aminé naturel,
R₂ est arginine (R), histidine (H), acide glutamique, cystéine, sérine ou de préférence acide aspartique (D),
R₃ est histidine (H), phénylalanine (F) ou tyrosine (Y),
R₄ est phénylalanine (F), glutamine (Q) ou leucine (L), et
F est phénylalanine et W est tryptophane ; ou
(ii) un fragment du peptide (I) comprenant au moins les huit acides aminés consécutifs
F-X₂-R₂-R₃-W-X₃-X₄-R₄ (Ib)
provenant de la séquence de formule (I)
où le peptide (I) ou (Ib) est modifié par substitution de façon que :
R₂, X₃ et/ou X₄ soient, indépendamment les uns des autres, remplacés par un résidu d'acide aminé α, α-disubstitué de l'acide α-aminoisobutyrique, de l'acide 1-amino-cyclopropane-1-carboxylique, de l'acide 1-amino-cyclopentane-1-carboxylique, de l'acide 1-amino-cyclohexane-1-carboxylique, de l'acide 4-amino-pipéridine-4-carboxylique ou de l'acide 1-amino-cycloheptane-1-carboxylique,
et où la modification est telle qu'une conformation en hélice α dans le peptide soit induite, augmentée ou maintenue.

2. Composé selon la revendication 1, où MDM2 est DM2 de l'humain.

3. Composé selon toute revendication précédente, où p53 est p53 de l'humain.

4. Composé selon toute revendication précédente, où R₂ est l'acide α-aminoisobutyrique (Aib), X₃ est l'acide α-aminoisobutyrique et/ou X₄ est l'acide 1-amino-cyclopropane-1-carboxylique (Ac₃c).

5. Composé selon l'une quelconque des revendications précédentes consistant en pas plus de 15 acides aminés (15 mères).

6. Composé selon l'une quelconque des revendications précédentes, où le peptide comprenant un motif d'acides aminés de la formule (I) est sélectionné dans le groupe consistant en peptides ayant les séquences M-P-R-F-M-D-Y-W-E-G-L-N, Q-P-T-F-S-D-Y-W-K-K-L-L-P et P-X-F-X-D-Y-W-X-X-L.

7. Composé selon l'une quelconque des revendications 1 à 5, où pour
F-X₂-R₂-R₃-W-X₃-X₄-R₄ (II)
X₂ est méthionine, isoleucine, thréonine, arginine, alanine ou sérine, de preference méthionine ;
X₃ est acide glutamique, thréonine, alanine, phénylalanine ou sérine, de préférence acide glutamique ; et
X₄ est glycine, glutamine, thréonine, alanine ou acide aspartique, de préférence glycine.

8. Composé selon l'une quelconque des revendications 1 à 6, où le fragment a pour formule
X₁-F-X₂-R₂-R₃-W-X₃-X₄-R₄ (Ic),
où R₁, R₂, R₃, R₄ indépendamment les uns des autres ont chacun les significations et les préférences données pour la formule (I),
X₁ est arginine, asparagine, alanine, thréonine ou valine ; en particulier arginine
X₂ est méthionine, isoleucine, thréonine, arginine, alanine ou sérine ; de préférence méthionine ;
X₃ est acide glutamique, thréonine, alanine, phénylalanine ou sérine ; de préférence acide glutamique ; et
X₄ est glycine, glutamine, thréonine, alanine ou acide aspartique, de préférence glycine.

9. Composé de toute revendication précédente un ou plusieurs groupes protecteurs.

10. Composé de toute revendication précédente portant sous la forme d'un sel.

11. Composé de toute revendication précédente où le peptide est sous forme cyclique.

12. Composé selon la revendication 11, qui contient un pont disulfure, un pont thioéther ou un lactame.

13. Conjugué comprenant le composé de toute revendication précédente, attaché de manière covalente à un autre peptide ou une autre protéine.

14. Conjugué comprenant un composé de l'une quelconque des revendications 1 à 12, marqué directement ou par un espaceur ou un groupe linker avec une enzyme, un marqueur fluorescent, un marqueur chimioluminescent, un chélate de métal, des particules paramagnétiques ou de la biotine.

15. Utilisation d'un composé ou conjugué selon toute revendication précédente pour l'identification d'une molécule se liant à MDM2.

16. Utilisation d'un composé ou conjugué selon l'une quelconque des revendications 1 à 14 pour la purification d'un partenaire de liaison en particulier MDM2.

17. Utilisation d'un composé ou conjugué selon l'une quelconque des revendications 1 à 14 dans une méthode ayant pour but l'identification ou la conception de composés qui interfèrent avec la liaison de MDM2 à p53.

18. Utilisation d'un composé ou conjugué selon l'une quelconque des revendications 1 à 14 pour le diagnostic *in vitro* d'une maladie.

19. Composition pharmaceutique qui est appropriée pour l'administration à un animal à sang chaud, comprenant des humains, ou à des cellules ou lignées de cellules dérivables d'un animal à sang chaud, comprenant un humain, pour le traitement ou la prévention d'une maladie qui répond à l'inhibition de l'interaction de p53 avec MDM2, ladite composition comprenant une quantité d'un composé ou conjugué selon l'une quelconque des revendications 1 à 14, qui est efficace pour ladite inhibition, en même temps qu'un ou plusieurs supports pharmaceutiquement acceptables.

20. Procédé pour la préparation d'un composé ou d'un conjugué selon l'une quelconque des revendications 1 à 14, comprenant la réaction d'un fragment d'un tel peptide, qui a un groupe carboxy libre, ou son dérivé réactif, avec un fragment complémentaire qui a un groupe amino avec au moins un groupe un atome d'hydrogène libre ou avec son dérivé réactif ayant pour résultat la formation d'une liaison peptidique et, si on le souhaite, l'élimination d'un groupe protection présent ou la dérivatisation dudit peptide ou dérivé.

21. Composé ou conjugué selon l'une quelconque des revendications 1 à 14 à utiliser dans une méthode de traitement.

22. Composé ou conjugué selon la revendication 21, où le traitement d'une maladie hyperproliférative.

23. Composé ou conjugué selon la revendication 22 où le traitement est une tumeur.

24. Utilisation d'un composé ou conjugué selon l'une quelconque des revendications 1 à 14 pour la préparation d'une composition pharmaceutique pour le traitement ou la prévention d'une maladie hyperproliférative.

25. Utilisation selon la revendication 24, où le traitement est une tumeur.
